(19) Europäisches Patentamt
European Patent Office
Office européen des brevets

(11) Publication number: **0 002 512**
**B1**

(12) # EUROPEAN PATENT SPECIFICATION

(45) Date of publication of patent specification: **22.02.84**

(21) Application number: **78101622.5**

(22) Date of filing: **09.12.78**

(51) Int. Cl.³: **C 07 D 471/18,**
A 61 K 31/46 //C07D213/80,
C07D213/82, C07D221/16,
(C07D471/18, 221/00,
221/00, 209/00)

(54) **Imino-bridged benzocycloheptapyridines, process for their preparation and pharmaceutical composition thereof.**

(30) Priority: **15.12.77 US 860666**

(43) Date of publication of application:
**27.06.79 Bulletin 79/13**

(45) Publication of the grant of the patent:
**22.02.84 Bulletin 84/8**

(84) Designated Contracting States:
**BE CH DE FR GB LU NL SE**

(56) References cited:
**US - A - 3 892 756**

(73) Proprietor: **MERCK & CO. INC.**
**126, East Lincoln Avenue P.O. Box 2000**
**Rahway New Jersey 07065 (US)**

(72) Inventor: **Leroy, Shephard Kenneth**
**726 Marietta Drive**
**Ambler R.D. Pa 19002 (US)**
Inventor: **Halczenko, Wasyl**
**1003 Clymer Road**
**Hatfield Pa 19440 (US)**
Inventor: **Gordon, Brenner Daniel**
**11 Pitcher Avenue**
**Medford Ma 02155 (US)**

(74) Representative: **Blum, Rudolf Emil Ernst et al,**
**c/o E. Blum & Co Patentanwälte Vorderberg 11**
**CH-8044 Zürich (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 002 512 B1

Imino-bridged benzocycloheptapyridines, process for their preparation and pharmaceutical composition thereof.

BACKGROUND OF THE INVENTION

This invention is concerned with novel benzocycloheptapyridines with an imine bridge in the cycloheptane ring, derivatives and pharmaceutically acceptable salts thereof which are useful as anti-anxiety agents, muscle relaxants, and in the treatment of extrapyramidal disorders such as in Parkinson's disease.

Structurally related compounds are known in the art to have qualitatively similar utilities. For example U.S. Patent 3,892,756 discloses 10,11-dihydro-5H-dibenzo[a,d]cyclohepten-5,10-imine and derivatives; and Belgian Patent 829,075 discloses 9,10-dihydroanthracen-9,10-imines and derivatives.

It is an object of this invention to provide the novel compounds, novel processes for their synthesis, and pharmaceutical compositions comprising them as active ingredient.

DETAILED DESCRIPTION OF THE INVENTION:

The novel compounds of this invention have the structural formula:

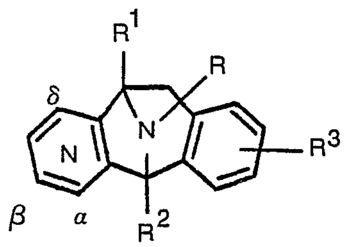

or

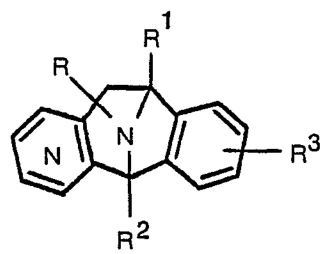

or they are pharmaceutically acceptable salts of said substances,
wherein
$R^1$ and $R^2$ are independently
- (1) hydrogen
- (2) an alkyl group having 1—5 carbon atoms,
- (3) an alkenyl group having 2—5 carbon atoms,
- (4) a phenylalkyl group having 1—3 carbon atoms in the alkyl moiety,
- (5) a cycloalkyl group having 3—6 carbon atoms,
- (6) a cycloalkylalkyl group having 3—6 carbon atoms in the cycloalkyl moiety, and 1—3 carbon atoms in the alkyl moiety,

R is
- (1) $R^1$,
- (2) Phenyl, or
- (3) di($C_{1-3}$alkyl)amino-$C_{1-5}$alkyl, and

$R^3$ is
- (1) hydrogen,
- (2) halogen,
- (3) an alkoxy group having 1—5 carbon atoms,
- (4) trifluoromethylthio,
- (5) cyano, or
- (6) carboxy,

Preferred compounds of said structural formulae or pharmaceutically acceptable salts thereof are those, wherein
$R^1$ and $R^2$ are independently
- (1) hydrogen,
- (2) methyl or ethyl,
- (3) vinyl or allyl,
- (4) benzyl,
- (5) cyclopropyl or cyclohexyl,
- (6) a cycloalkylalkyl group having 3—6 carbon atoms in the cycloalkyl moiety, and 1—3 carbon atoms in the alkyl moeity;

R is
- (1) $R^1$,
- (2) phenyl, or
- (3) di($C_{1-3}$alkyl)amino-$C_{1-5}$alkyl, and

$R^3$ is
- (1) hydrogen,
- (2) chloro, bromo, fluoro or iodo,

(3) methoxy,
(4) trifluoromethylthio,
(5) cyano, or
(6) carboxy.

It is preferred that the nitrogen of the pyridine ring be in the $\alpha$, $\beta$ or $\delta$-position, and most preferably in the $\beta$-position.

Another preferred group of compounds is that wherein $R^3$ is hydrogen.

Where $R^3$ is other than hydrogen, it is preferred that is occupy the $\beta'$ and or $\gamma'$ positions of the tricyclic system.

Preferred definitions for R, $R^1$ and $R^2$ are hydrogen, alkyl having 1—5 carbon atoms, preferably methyl or benzyl. Especially preferred are the compounds, wherein $R^3$ is hydrogen, $R^1$ is hydrogen, and R and $R^2$ are independently hydrogen, alkyl having 1—5 carbon atoms or benzyl.

The novel compounds of this invention are generally preparable by formation of the imine bridge by addition of an amino group attached to the seven membered ring across a double bond, also in the seven membered ring. The reaction is accomplished by treating the amino compound in the presence of an acid until the ring closure is complete.

A further object of the present invention accordingly is a process for the preparation of a compound of structural formula:

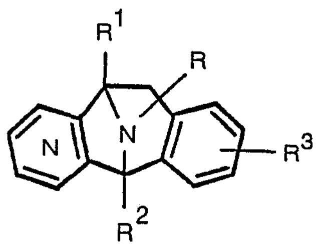

or

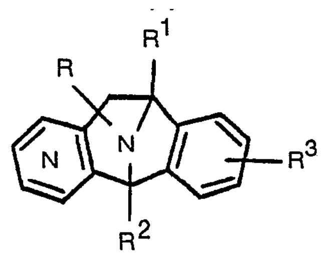

wherein
$R^1$, $R^2$, R and $R^3$ are as outlined before.

Said process comprises treating a compound of structural formula

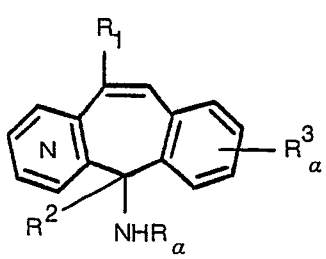

or

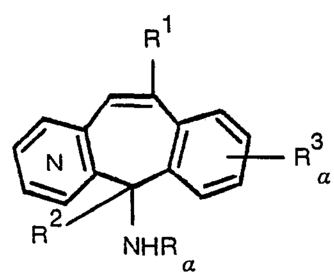

wherein

$R_\alpha$ is R, lower alkanoyl, phenyl-sulfonyl, tosyl, alkoxycarbonyl, diphenylphosphinyl or arylsulfonyl, and

$R^3_\alpha$ is hydrogen or halogen,

with acid to produce the compound of structural formula:

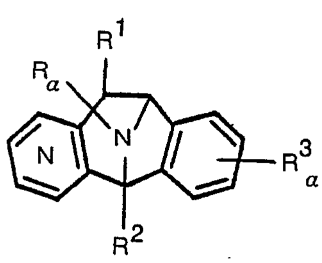

or

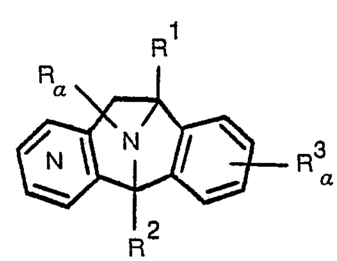

followed by hydrolysis or hydrogenolysis of $R_\alpha$, where $R_\alpha$ is other than R, and which optionally comprises a further treatment of the compounds, in which $R^3_\alpha$ is bromo, with either

(a) a corresponding alkali metal alkoxide to produce the compound wherein $R^3$ is alkoxy having 1—5 carbon atoms, or

(b) trifluoromethylthio-copper to produce the compound wherein $R^3$ is trifluoromethyl, or

(c) cuprous cyanide to produce the compound wherein $R^3$ is cyano, and which optionally comprises

(d) a following hydrolysis of the compound resulting from step (c), in which $R^3$ is cyano to produce the compound wherein $R^3$ is carboxy.

The treatment of the amino compound with acid is preferably accomplished in an inert solvent such as a lower alkanol such as ethanol, propanol, butanol, or a chlorinated hydrocarbon such as chloroform.

Reaction times of a few minutes to several days are employed depending on the structure of the particular starting material.

Temperatures from about 15°C. to about 150°C. or reflux temperatures can be employed. Where $R^1$ is other than hydrogen, the reaction proceeds readily at the lower temperatures. The acid can be a hydrogen halide or other anhydrous inorganic or organic acid such as trifluoroacetic acid, and where $R^1$ is other than hydrogen, chromatography of the starting material on silica gel with chloroform can be sufficiently acidic to cause the cyclization.

If the nitrogen of the ring closed material carries protecting groups such as lower alkanoyl, alkoxycarbonyl, phenyl-sulfonyl, tosyl or diphenylphosphinyl, these are removed by hydrolysis or hydrogenolysis. An acid or base hydrolysis can be performed at 25° to about 100°C. for 2 to 48 hours, preferably 6 to about 24 hours. In the case of the aryl-sulfonyl protective groups they can also be removed by hydrogenolysis conveniently with an excess of sodium bis(2-methoxyethyl) aluminum hydride in an inert organic solvent such as toluene at about 15°C. to about 50°C., preferably at 20° to about 30°C. for 6—48 hours, preferably about 12—24 hours.

An alternative procedure of forming the imine bridge comprises treating a compound with a bromo or chloro and an oxo group in the seven-membered ring with an amine preferably at about 10°C. to about 20°C. in an inert organic solvent such as tetrahydrofuran, dimethoxyethane, acetonitrile, DMF or the like for 5 to about 36 hours. This results in displacement of the bromo group by the amino group followed by spontaneous addition across the oxo function. The hydroxyl function that is formed in the reaction is converted, to a chloro group by treatment with thionyl chloride in an inert solvent such as benzene, toluene or the like at 10°C. to about 100°C. for 15 minutes to about 2 hours. The resulting compound of formula:

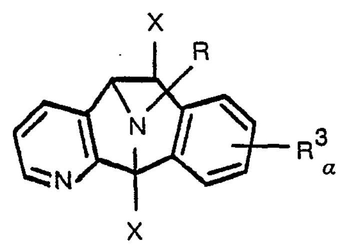

is then hydrogenolyzed.

A further object of the present invention accordingly is a process for the preparation of a compound of structural formula:

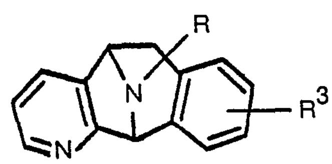

wherein

R is

(1) hydrogen
(2) an alkyl group having 1—5 carbon atoms,
(3) an alkenyl group having 2—5 carbon atoms,
(4) a phenylalkyl group having 1—3 carbon atoms in the alkyl moiety,
(5) a cycloalkyl group having 3—6 carbon atoms,
(6) a cycloalkylalkyl group having 3—6 carbon atoms in the cycloalkyl moiety, and 1—3 carbon atoms in the alkyl moiety, or
(7) di($C_{1-3}$alkyl)amino-$C_{1-5}$alkyl, and

$R^3$ is

(1) hydrogen,
(2) halogen,
(3) an alkoxy group having 1—5 carbon atoms,
(4) trifluoromethylthio,
(5) cyano, or
(6) carboxy,

which comprises hydrogenolysis of the X-groups in a compound of formula:

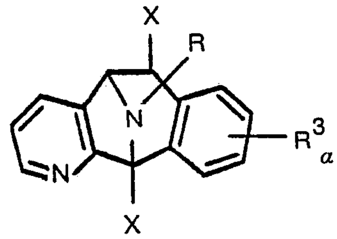

wherein

X is Br or Cl, and

$R_{\alpha}^{3}$ is hydrogen or halogen,

to produce a compound of formula

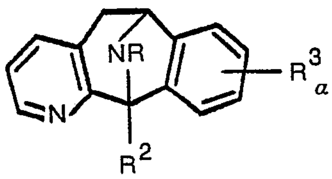

and comprises optionally a further treatment of the compound, wherein $R_{\alpha}^{3}$ is bromo, with either

(a) either a corresponding alkali metal alkoxide to produce the compound wherein $R^3$ is an alkoxy group having 1—5 carbon atoms, or with

(b) trifluoromethylthio-copper to produce the compound wherein $R^3$ is trifluoromethyl, or

(c) cuprous cyanide to produce the compound wherein $R^3$ is cyano, and comprises optionally

(d) a further hydrolysis of the compounds of step (c), wherein $R^3$ is cyano to produce the compound wherein $R^3$ is carboxy.

The hydrogenolysis can be a catalytic hydrogenation, such as with 2,81—3,52 kg/cm² (40 to 50 p.s.i.) of hydrogen in the presence of a palladium, platinum, or the like, hydrogenation catalyst at about 10°C. to about 30°C. until the theoretical amount of hydrogen is consumed.

Alternatively, particularly wherein $R^3$ is halo the hydrogenolysis can be accomplished with a complex metal hydride such as lithium aluminum hydride or sodium borohydride.

The novel compounds of this invention wherein R is hydrogen are generally prepared by reduction of the N-hydroxy analog.

A further object of the present invention accordingly is a process for the preparation of a compound of structural formula:

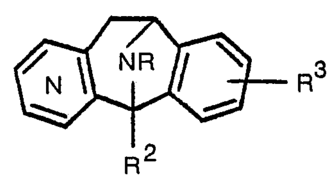

wherein $R^2$, $R^3$ and R are as defined before which comprises

(a) a reduction of a compound of formula

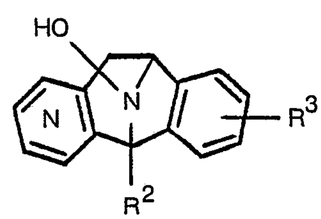

to produce the compound wherein R is hydrogen, followed optionally by

(b) an introduction of the radical R in the meaning of an alkyl group having 1—5 carbon atoms, an alkenyl group having 2—5 carbon atoms, a phenylalkyl group having 1—3 carbon atoms in the alkyl moiety, a cycloalkyl group having 3—6 carbon atoms, a cycloalkylalkyl group having 3—6 carbon atoms in the cycloalkyl moiety and 1—3 carbon atoms in the alkyl moiety, a phenyl group of a di($C_{1-3}$alkyl)amino-$C_{1-5}$alkyl into the compounds of formula:

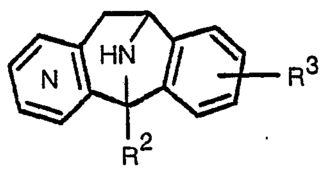

according to the treatment of the N-alkylation to produce the compound wherein R has the above outlined meaning and/or optionally

(c) a treatment of the compound wherein $R^3$ is bromo with a corresponding sodium or potassium alkoxide, with cuprous cyanide or with bis(trifluoromethylthio) mercury and copper dust to produce the compounds wherein $R^3$ is alkoxy having 1—5 carbon atoms, cyano or trifluoromethylthio respectively; and optionally comprising

(d) a following hydrolysis of the compound resulting from step (c), wherein $R^3$ is cyano, to produce the compound wherein $R^3$ is carboxy.

The preferred reducing agent is nascent hydrogen generated by the action of a metal, preferably zinc with an acid such as acetic acid at 40 to 100°C. for to about 10 hours.

Where R is other than hydrogen, the novel compounds may be prepared by introducing radical R into these compounds wherein R is hydrogen, preferably with the appropriate reagent of formula R-halo wherein halo represents chloro, bromo or iodo. The reaction is normally conducted in an inert solvent such as benzene, or toluene. However, the reagent R-halo, depending on its physical properties, may be used in sufficiently excess amount to act as solvent. It is preferred to conduct the reaction in the presence of an acid acceptor such as an inorganic carbonate such as sodium carbonate, an organic base such as pyridine, or a basic resin. Temperatures of about 50°C. to about 100°C. may be employed over reaction times of about 10 hours to about 5 days.

Where R is alkyl or substituted alkyl, the compounds also may be prepared by reduction of an N-acyl compound such as alkoxycarbonyl to give methyl or other alkanoyl groups to provide the other alkyl groups. The preferred reducing system is a metal hydride such as lithium aluminum hydride in an ethereal solvent such as ether, tetrahydrofuran or 1,2-dimethoxyethane. The reaction proceeds satisfactorily at room temperature but temperatures from about 0°C. to about 50°C. are appropriate with reaction times of 10—13 hours.

An additional alkylation method involves the treatment of the unsubstituted imine with an aldehyde and sodium cyanoborohydride ($NaCNBH_3$) in an ether such as tetrahydrofuran, 1,2-dimethoxyethane or di(2-methoxyethyl)ether, preferably tetrahydrofuran, at about 10°—50°C., preferably 25°C., until the reaction is substantially complete, usually for about 6 hours to about 3 days, preferably about 2 days.

Novel compounds having a substituent on the benzenoid ring are generally prepared by metathesis of the appropriate bromo or iodo compound. For example treatment with an alkali metal lower alkoxide such as a sodium lower alkoxide in the presence of copper dust in a solvent such as dimethyl formamide at 50—150°C. for 1—10 hours yields the corresponding lower alkoxy compound.

Similarly treatment of a bromo or iodo compound with cuprous cyanide in a solvent such as dimethyl formamide at reflux temperature for 1—10 hours yields the corresponding cyano compound.

Hydrolysis of the above cyano compounds with a mineral acid such as hydrochloric acid at reflux temperature produces the corresponding carboxy substituted compounds.

Also treatment of the bromo or iodo compounds with bis(trifluoromethylthio)mercury and copper dust or trifluoromethylthio-copper in a solvent such as dimethyl formamide or hexamethylphosphoric acid triamide at about 100—200°C. for 1—10 hours yields the trifluoromethylthio derivatives.

Thus there are prepared the compounds:

9-$R^3$-5,6-dihydro-6,12-dimethylbenzo[5,6]cyclohepta[1,2-c]pyridin-6,11-imine, and 9-$R^3$-5,6-dihydro-6-methylbenzo[5,6]cyclohepta[1,2-c]pyridin-6,11-imine, wherein $R^3$ is lower alkoxy, cyano, carboxy and trifluoromethylthio.

The starting materials and processes used for preparing the intermediates used in the above described processes are fully described in the Examples.

Also included within the scope of the present invention are the non-toxic pharmaceutically acceptable salts of the novel compounds. Acid addition salts of the imine compounds are formed by mixing a solution of the imine with a solution of a pharmaceutically acceptable non-toxic acid such as hydrochloric acid, fumaric acid, maleic acid, succinic acid, acetic acid, or the like. Where the novel compound carries a carboxylic acid group, the invention also contemplates sodium, potassium, and calcium salts thereof.

If in the inventive compounds having the stated formulae, $R^3$ is a hydrogen atom, those which are preferred are those having the following structural formula:

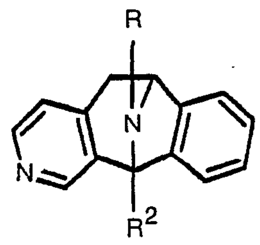

wherein

R is hydrogen, alkyl having 1—5 carbon atoms or benzyl, and

$R^2$ is hydrogen or alkyl having 1—5 carbon atoms.

The present invention furthermore concerns a pharmaceutical composition comprising a pharmaceutical carrier and an effective amount of a compound of formula:

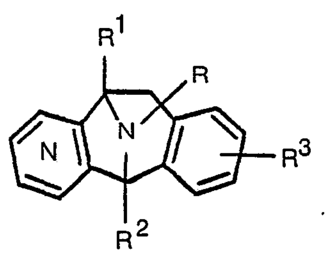

or

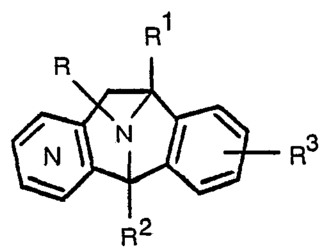

or a pharmaceutically acceptable salt thereof, wherein R, $R^1$, $R^2$ and $R^3$ are defined before.

The novel compounds can be resolved into their optical isomers by standard techniques, such as the formation of diastereomeric pairs by salt formation with an optically active acid, such as (—)-di-p-toluoyl-d-tartaric acid and/or (+)-di-p-toluoyl-1-tartaric acid followed by fractional crystallization and regeneration of the free base.

The novel imines of the present invention are capable of producing anxiety relief without causing excessive sedation or sleep at a dosage level of from about 0.01 to about 10 mg. per kilogram of body weight preferably about 0.5—5 mg/kg. of body weight on a regimen of 1—4 times a day. In addition, the novel compounds of the present invention are useful as muscle relaxants, anticonvulsants and in the treatment of extrapyramidal disorders when indicated at comparable dosage levels. It is understood that the exact treatment level will depend upon the case history of the animal or human individual being treated and in the last analysis the precise treatment level falling within the above guidelines is left to the discretion of the therapist.

Also included within the scope of the present invention are pharmaceutical compositions comprising the imines of this invention. Preferably these compositions are in unit dosage forms such as tablets, pills, capsules, powders, granules, sterile parenteral solutions or suspensions, or suppositories for oral, parenteral or rectal administration. A unit dose contains from about 0.1 to about 500 mg. of active ingredient.

Example 1

5,6-Dihydro-6,12-dimethylbenzo[5,6]cyclohepta[1,2-c]pyridin-6,11-imine

*Step A:*

Preparation of *trans*-4-($\alpha$-methylstyryl) nicotinamide

t-Butyl alcohol (29.6 g.) is added to a suspension of sodium hydride (12.6 g., 57% oil dispersion) in dimethylformamide (300 ml.) and the resulting mixture is warmed on the steam bath until hydrogen evolution ceases. The resulting stirred solution is cooled to 0°C. and a solution of 23.6 g. of 4-methylnicotinonitrile in dimethylformamide (100 ml.) is added dropwise (0.5—1 hour). The mixture is stirred at this temperature for an additional hour and a solution of acetophenone (24 g.) in dimethylformamide (100 ml.) is added dropwise. After 24 hours, the reaction mixture is poured over ice and the solution is acidified by the addition of glacial acetic acid. The solid that separates is filtered, washed with water and dried to give 38 g. of *trans*-4-($\alpha$-methylstyryl)nicotinamide, m.p. 150—155°C. Recrystallization from ethyl acetate produces product of m.p. 153.5—155.5°C.

*Step B:*

Preparation of *trans*-4-($\alpha$-methylstyryl)nicotinic acid

A mixture of *trans*-4-($\alpha$-methylstyryl)nicotinamide (5.1 g.), potassium hydroxide (5 g.), ethanol (50 ml.), and water (50 ml.) is heated under reflux for 24 hours. The ethanol is removed by distillation and the resulting aqueous solution is chilled and acidified by the addition of glacial acetic acid (5 ml.). The white solid that separates is filtered, washed with water, and dried to give 4.46 g. of *trans*-4-($\alpha$-methylstyryl)nicotinic acid, m.p. 151—158°C. Recrystallization from ethanol followed by recrystallization from methanol gives material with m.p. 160—161°C.

*Step C:*

Preparation of 6-methylbenzo[5,6]cyclohepta[1,2-c]pyridin-11-one

*Trans*-4-($\alpha$-methylstyryl)nicotinic acid (7.77 g.) is added to polyphosphoric acid with stirring (210°C.). The temperature of the stirred mixture is raised to 225°C. and maintained at 225—230°C. for 0.25 hours. After cooling to 50°C. ice and water is added to 600 ml. total volume. The solution is made alkaline by the addition of concentrated aqueous ammonia and the solid that separates on cooling is filtered, washed with water, and dried to give 3.0 g. of 6-methylbenzo[5,6]cyclohepta[1,2-c]-pyridin-11-one, m.p. 112—116°C. Recrystallization from cyclohexane provides material with m.p. 117.5—119.5°C.

Employing the procedure substantially as described in Example 1, Steps A, B and C but optionally substituting for the acetophenone used in Step A thereof a corresponding molecular amount of a compound of structure

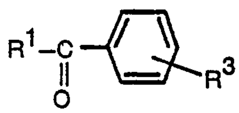

there are obtained the 6-$R^1$-benzo[5,6]cyclohepta[1,2-]pyridin-11-ones described in Table I in accordance with the following reaction scheme:

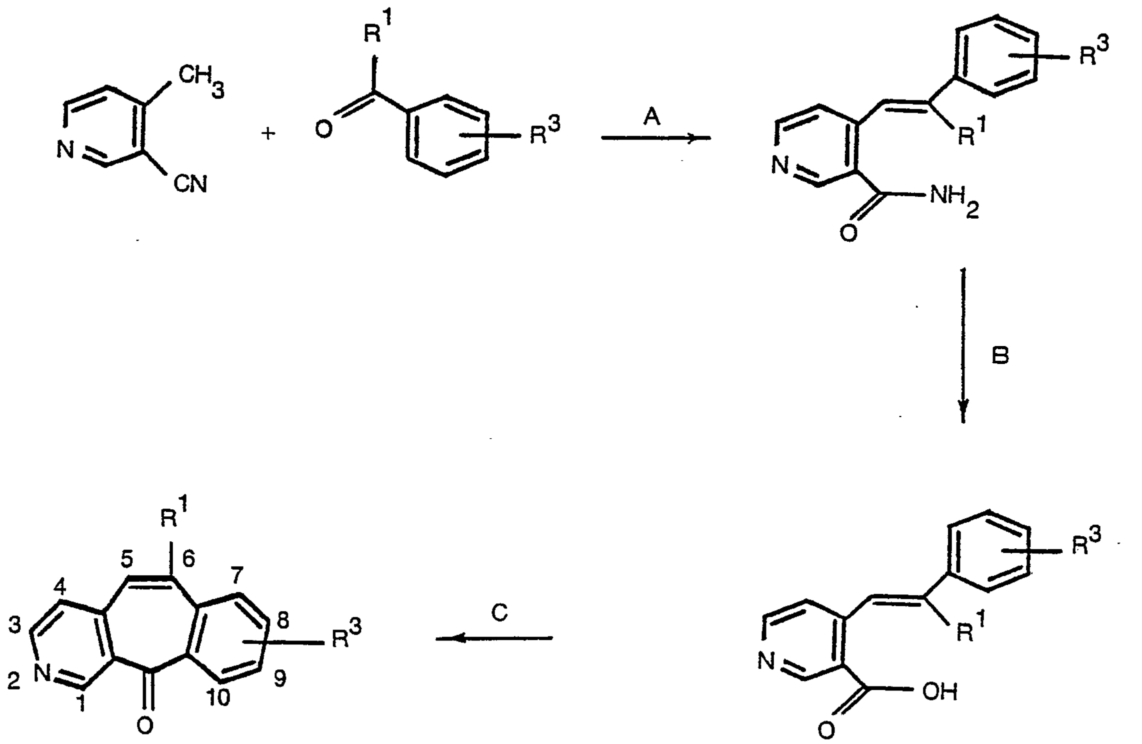

TABLE I

| $R^1$ | $R^3$ |
|---|---|
| $CH_3$— | 9-Br |
| n-$C_3H_7$— | 9-Br |
| $CH_2$=CH—$CH_2$— | H |
| 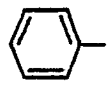 $CH_2$– | H |
| 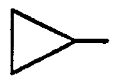 | 9-Br |
| 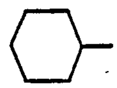 | H |
| 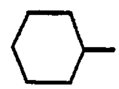 $CH_2$– | 9-Br |
| 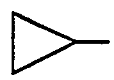 $CH_2$– | H |
| $C_2H_5$— | 9-F |
| $CH_3$— | 9-F |
| H | 9-Br |
| H | 9-F |

*Step D:*
Preparation of 6-methyl-11-methyliminobenzo[5,6]cyclohepta[1,2-c]pyridine

A solution of titanium tetrachloride (0.7 ml., 0.006 mole) in benzene (20 ml.) is added rapidly to a stirred solution of methylamine (1.24 g., 0.04 mole) and 6-methylbenzo[5,6]cyclohepta[1,2-c]pyridin-11-one (2.21 g., 0.01 mole) in benzene (150 ml.). After 4 hours, anhydrous potassium carbonate is added and the reaction mixture is filtered. The filtrate is evaporated to a pale yellow oil, (2.95 g.).

*Step E:*
Preparation of 5,6-dihydro-6,12-dimethylbenzo[5,6]cyclohepta[1,2-c]pyridin-6,11-imine

Sodium borohydride (0.20 g.) is added to a solution of 6-methyl-11-methyliminobenzo[5,6]-cyclohepta[1,2-c]pyridine (2.95 g.) in acetonitrile (100 ml.). The resulting mixture is heated on the steam bath for 4 hours. Another 0.10 g. of sodium borohydride is added and the mixture is heated for an additional two hours. The solvent is removed under reduced pressure and the residue is dissolved in 3*N* HCl (100 ml.). The acidic solution after extraction with ether is made alkaline by the addition of 40% NaOH solution and re-extracted with methylene chloride. After drying, the solvent is removed *in vacuo* to give an amber oil, (2 g.). This material is chromatographed over 50 g. of silica gel with $CHCl_3$ and increasing amounts of ethyl acetate. The produce is eluted with ethyl acetate and on evaporation yields a viscous oil, 1.2 g. Trituration with petroleum ether converts the oil to a white solid, m.p. 77—82°C.

Following the procedure substantially as described in Example 1, Steps D and E but substituting for the methylamine used in Step D, an equimolecular amount of a compound of formula R—$NH_2$ described in Table II, there are produced the 5,6-dihydro-6-$R^1$-12-R-benzo[5,6]cyclohepta[1,2-c]-pyridin-6,11-imines also described in Table II in accordance with the following scheme:

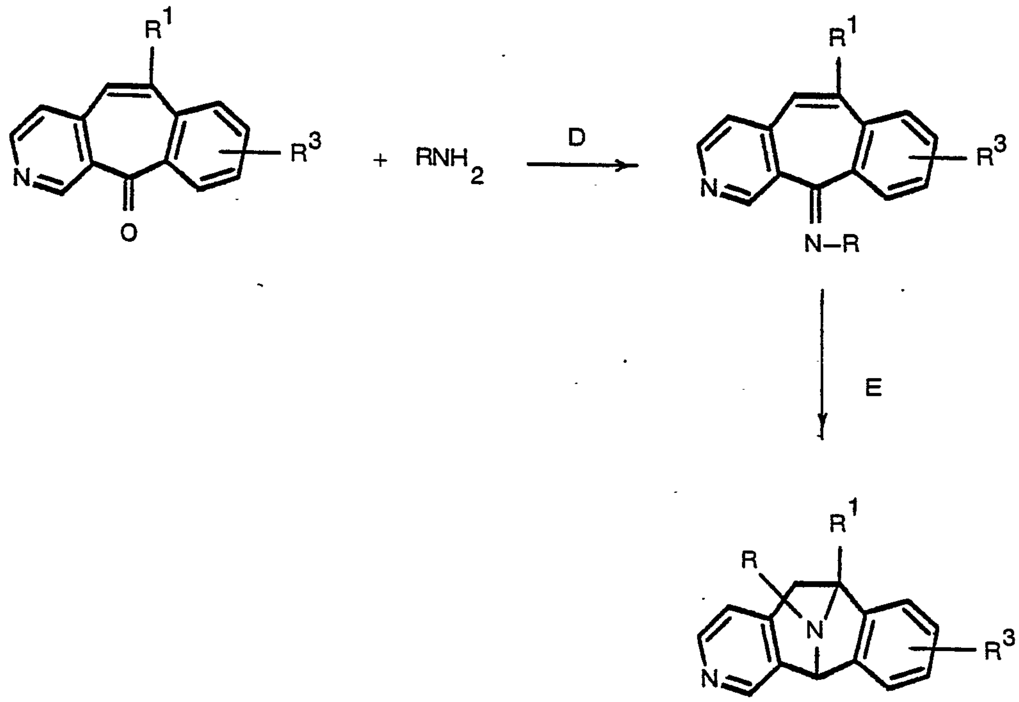


TABLE II

| $R^1$ | $R^3$ | R |
|---|---|---|
| $CH_3-$ | 9-Br | $-CH_3$ |
| n-$C_3H_7$ | 9-Br | $-CH_2-CH=CH_2$ |
| $CH_2=CH-CH_2-$ | H | $-CH_2-$ 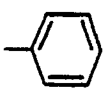 |
| 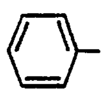 $CH_2-$ | H | 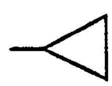 |
| 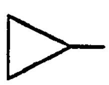 | 9-Br | $-CH_3$ |
| 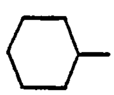 | H | $-CH_2-$ 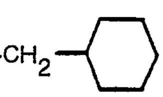 |
| 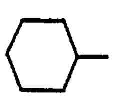 $CH_2-$ | 9-Br | $-CH_2-$ 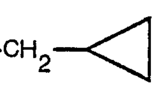 |
| 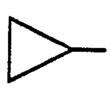 $CH_2-$ | H | 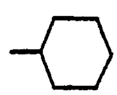 |
| $C_2H_5$ | 9-F | $-CH_3$ |
| $CH_3-$ | 9-F | $-CH_3$ |
| $CH_3-$ | H | $-CH_2CH_2N(CH_3)_2$ |
| $CH_3-$ | H | $-CH_2CH_2CH_2N(CH_3)_2$ |

Example 2

5,6-Dihydro-12-methylbenzo[5,6]cyclohepta[1,2-c]pyridin-6,11-imine dihydrochloride, hydrate

*Step A:*

Preparation of 11-methylaminobenzo[5,6]cyclohepta[1,2-c]pyridine hydrochloride

Methylamine (6.2 g., 0.2 mole) is added to a mechanically stirred mixture of benzo[5,6]cyclohepta[1,2-c]pyridin-11-one (10 g., 0.048 mole) in benzene (1000 ml.). A solution of $TiCl_4$ (3.5 ml.) in benzene (100 ml.) is added and the mixture is stirred at 25°C. for 18—24 hours. Anhydrous potassium carbonate is added to the mixture and it is filtered through diatomaceous earth. The filtrate is stripped to an amber oil and this oil is dissolved in acetonitrile (500 ml.). Sodium borohydride (3 g.) is added and the mixture stirred at 25°C. for 18 hours. An additional 0.5 g. of $NaBH_4$ is added and the mixture is warmed on the steam bath for one hour. Dilute HCl (3*N*) is added to the cooled reaction mixture and it is evaporated to dryness under reduced pressure. The residue is dissolved in 3*N* HCl (200 ml.), treated with decolorizing carbon and filtered. The filtrate is extracted with ether and the aqueous filtrate is made alkaline by the addition of 20% NaOH solution. The basic material is extracted into $CHCl_3$ and concentrated to an amber oil. The oil is dissolved in 50 ml. of ethanol and treated with 11*N* HCl to give 7.75 g. of 11-methylaminobenzo[5,6]cyclohepta[1,2-c]pyridine hydrochloride, m.p. 216—221°C.

*Step B:*

Preparation of 5,6-dihydro-12-methylbenzo[5,6]cyclohepta[1,2-c]pyridin-6,11-imine dihydrochloride hydrate

11-Methylaminobenzo[5,6]cyclohepta[1,2-c] pyridine hydrochloride is treated with a boiling mixture of methanol and 2-propanol (50:50, 750 ml.) and evaporated to a volume of 250 ml. The solid that separates is filtered, 2.16 g., m.p. 221—223°C. dec. The abbreviation "dec." in this specification means "decomposition". Spectral investigation indicates this solid to be a mixture of the hydrochloride of 11-methylaminobenzo[5,6]cyclohepta[1,2-c]pyridine and the title compound. The filtrate is evaporated *in vacuo* and the residue is treated with ethanol (25 ml.) and the solid that separates on standing is collected and dried to give 1.9 g. of product, m.p. 211—215°C. Recrystallization of this solid from ethanol gives 5,6-dihydro-12-methylbenzo[5,6]cyclohepta[1,2-c]pyridin-6,11-imine dihydrochloride hydrate, m.p. 216.5—218.5°C.

Employing the procedure substantially as described in Example 2, Steps A and B but substituting for the methylamine used in Step A, an equimolecular amount of an amine of formula R—$NH_2$, wherein R is

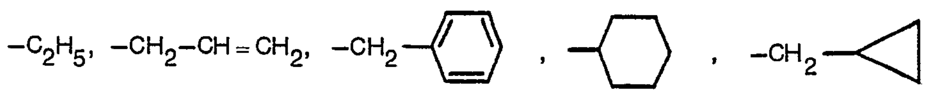

or —$CH_2CH_2N(CH_3)_2$, there is produced respectively a 5,6-dihydro-12-R-benzo[5,6]cyclohepta[1,2-c]-pyridin-6,11-imine, of formula

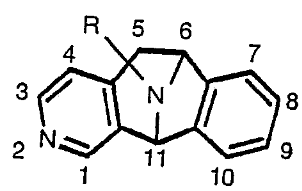

wherein R is as defined immediately above.

Example 3

11-Methyl-5,6-dihydrobenzo[5,6]cyclohepta[1,2-c]pyridin-6,11-imine and hydrogen fumarate salt

*Step A:*

Preparation of 5-bromobenzo[5,6]cyclohepta[1,2-c]pyridin-11-one

Bromine (4.18 ml.) in acetic acid (50 ml.) is added dropwise to a stirred solution of benzo[5,6]cyclohepta[1,2-c]pyridin-11-one (12.35 g.) in acetic acid (250 ml.). The resulting mixture is heated on the steam bath for 24 hours and the acetic acid is removed under reduced pressure. Saturated $Na_2CO_3$ solution (500 ml.) is added to the residue and the deep yellow solid that forms is collected, washed with water, and dried. This crude product is recrystallized from 1-chlorobutane to give 9.3 g., m.p. 167—169°C.

*Step B:*

Preparation of 5-bromo-11-methylbenzo[5,6]cyclohepta[1,2-c]pyridin-11-ol

Methyl magnesium bromide (27.6 ml., 2.9 M in ether) is added dropwise to a solution of 5-bromobenzo[5,6]cyclohepta[1,2-c]pyridin-11-one (20.02 g., 0.07 mol) in dry THF (300 ml.) with cooling. When addition is complete, the cooling bath is removed and the mixture is stirred for 20—24 hours. The solvent is removed *in vacuo*, the residue is taken up in water (300 ml.) and acidified by the addition of acetic acid. The solid that forms is collected, washed with water then recrystallized from 2-propanol to give 16.25 g., m.p. 226.5—229.5°C.

Employing the procedure substantially as described in Step B, but substituting for the 5-bromo compound used as starting material, the 6-methyl compound from Example 1, Step C, there is produced 6,11-dimethylbenzo[5,6]cyclohepta[1,2-c]pyridin-11-ol, m.p. 200—205°C.

*Step C:*
Preparation of 5-bromo-11-methylenebenzo[5,6]cyclohepta[1,2-c]pyridine

The carbinol (6.1 g.) from Step B is dissolved in acetic acid (50 ml.) and concentrated $H_2SO_4$ (15 ml.) is added gradually with stirring. After 24 hours the reaction mixture is diluted with $H_2O$ (500 ml.) and made alkaline by the addition of 20% weight per volume NaOH solution. The basic mixture is extracted with ethyl acetate and the combined organic extracts are washed with $H_2O$, saturated NaCl solution, and dried (anhydrous $Na_2SO_4$). Removal of the solvent gives 5.65 g. of a dark brown oil.

*Step D:*
Preparation of 6-(4-methylpiperazinyl)-11-methylenebenzo[5,6]cyclohepta[1,2-c]pyridine

A mixture of 5-bromo-11-methylenebenzo[5,6]cyclohepta[1,2-c]pyridine (11.0 g.), 1-methylpiperazine (7.8 ml.), potassium tert.-butoxide (8.4 g.) and tert.-butanol (150 ml.) is heated under reflux for 4 hours. The tert.-butanol is removed *in vacuo* and the residue is taken up in methylene chloride (600 ml.). The resulting mixture is washed with 10% weight per volume NaCl solution, saturated NaCl solution, and dried (anhydrous $Na_2SO_4$). Filtration and removal of the solvent gives 10.76 g. of dark amber oil.

*Step E:*
Preparation of 11-methylenebenzo[5,6]cyclohepta[1,2-c]pyridin-6-one

A mixture of the product from Step D (10.76 g.) and 3*N* HCl (150 ml.) is stirred at room temperature for approximately 24 hours. The dark brown solution is made alkaline by the addition of 20% NaOH solution and extracted with methylene chloride. The extracts are washed with $H_2O$, saturated NaCl solution and dried ($Na_2SO_4$). Removal of the solvent *in vacuo* followed by trituration with petroleum ether (30—60%) provides 6.2 g., m.p. 98—110°C.

*Step F:*
Preparation of 11-methylenebenzo[5,6]cyclohepta[1,2-c]pyridin-6-one oxime

A mixture of the ketone from Step E (6.2 g.), anhydrous sodium acetate (3.1 g.), hydroxylamine hydrochloride (2.48 g.) and $CH_3OH$ (130 ml.) is heated under reflux for 24 hours. The solvent is removed *in vacuo*, water (100 ml.) is added to the residue and the resulting yellow-orange solid is collected and dried, 6.4 g., m.p. 183—205°C. Recrystallization from toluene provides material with m.p. 218.5—221°C. (dec.).

*Step G:*
Preparation of N-(5-6-dihydro-11-methylenebenzo[5,6]cyclohepta[1,2-c]pyridin-6-yl)hydroxylamine

A 10% HCl solution (50 ml.) is added dropwise to a cold (0—5°C.) solution of the oxime from Step F (2.6 g.), $NaCNBH_3$ (5.0 g.), ethanol (50 ml.) and THF (100 ml.). When the acid addition is complete, the cooling bath is removed and the resulting mixture is stirred overnight. The reaction mixture is diluted with $H_2O$ and extracted with ethyl acetate to remove unreacted oxime (recovery 0.7 g.). The resulting aqueous layer is brought to pH 8 with dilute NaOH solution and re-extracted with ethyl acetate. The combined extracts from the basic solution are washed ($H_2O$, saturated NaCl solution) and dried ($Na_2SO_4$). Removal of the solvent after filtration gives 1.7 g., yellow solid, m.p. 130—136°C. Recrystallization from ethyl acetate gives material with m.p. 138—141°C.

*Step H:*
Preparation of 12-hydroxy-11-methylbenzo[5,6]cyclohepta[1,2-c]pyridin-6,11-imine

A mixture of the N-substituted hydroxylamine from Step G (1.8 g.) and toluene (40 ml.) is heated to reflux for four and one-half hours. The solvent is removed under reduced pressure and the residue is chromatographed over silica gel. After removal of some by-products with methylene chloride (1)/ethyl acetate (1), the product is eluted with ethyl acetate. Removal of the solvent gives 1.4 g. of light yellow flakes.

*Step I:*
Preparation of 5,6-dihydro-11-methylbenzo[5,6]cyclohepta[1,2-c]pyridin-6,11-imine hydrogen fumarate

A mixture of the product from Step H (1.4 g.), zinc dust (1.4 g.), and acetic acid (40 ml.) is heated

to 80—100°C. for 4 hours. The reaction mixture is cooled, diluted with $H_2O$ (300 ml.) and filtered. The filtrate is made alkaline with 20% NaOH solution and extracted with ethyl acetate. The ethyl acetate extracts are washed with saturated NaCl solution and dried ($Na_2SO_4$). Removal of the solvent gives 1.1 g. of a straw-colored foam. This material is converted to its fumarate salt by treatment with 1.1 g. of fumaric acid in 150 ml. of boiling acetone, and gives 1.3 g., of 5,6-dihydro-11-methylbenzo[5,6]cyclohepta[1,2-c]pyridin-6,11-imine hydrogen fumarate, m.p. 194—195.5°C.

Employing the procedure substantially as described in Example 3, but using as starting material, benzo[5,6]cyclohepta[1,2-c]pyridin-11-one or the 9-bromo or 9-fluoro derivative thereof and using a Grignard reagent of formula $R^2MgX$ wherein X is Br or Cl, in Step B there are produced the 11-$R^2$-5,6-dihydrobenzo[5,6]cyclohepta[1,2-c]pyridin-6,11-imines described in Table III by the following process:

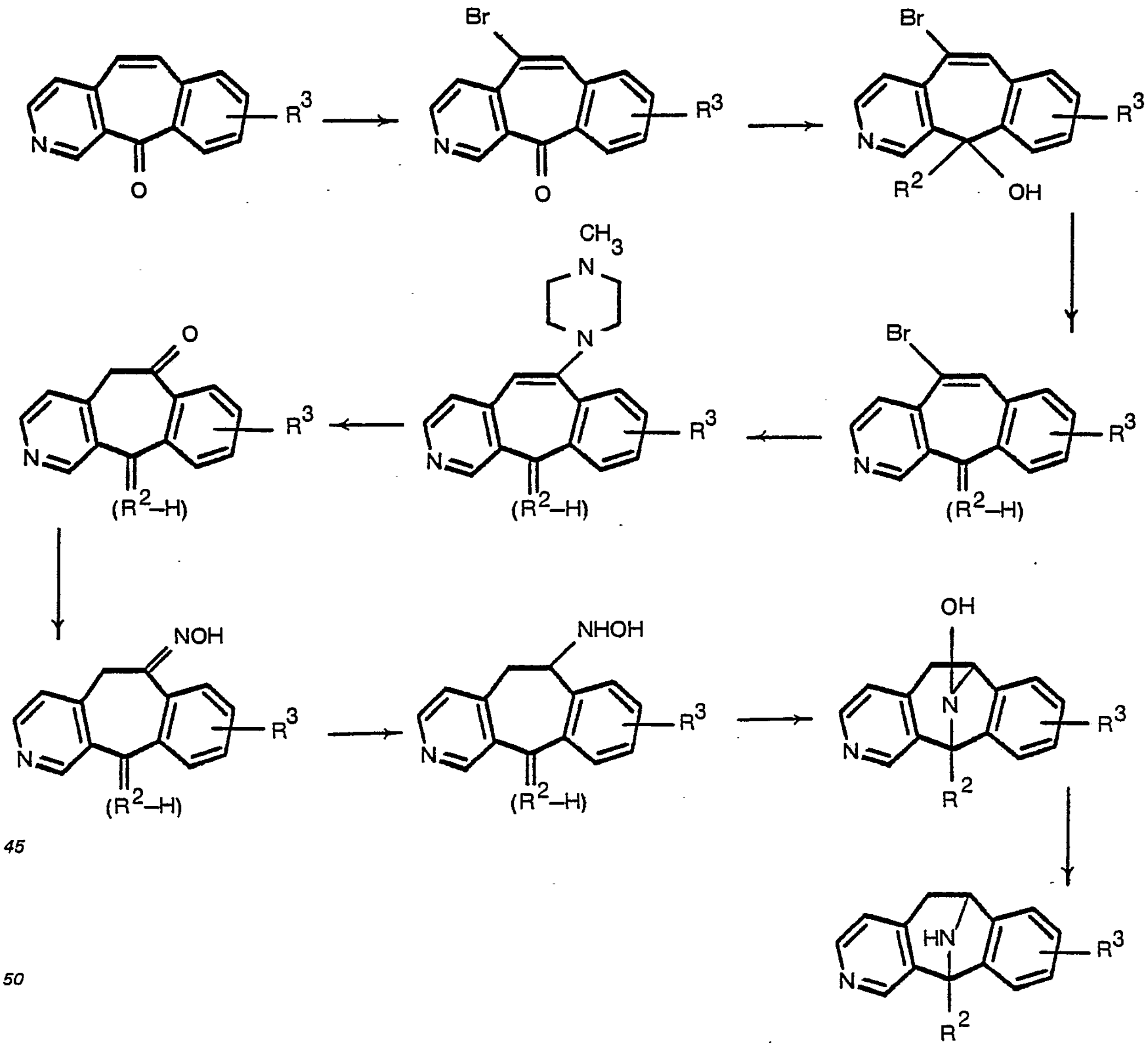

TABLE III

| $R^2$ | $R^3$ |
|---|---|
| $CH_3$— | 9-Br |
| $CH_3$— | 9-F |
| $C_2H_5$— | H |
| $CH_2$=$CHCH_2$— | 9-F |
| 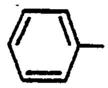 $CH_2$– | H |
| 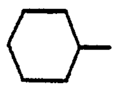 | 9-Br |
| 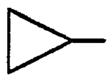 | H |
| 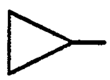 $CH_2$– | 9-Br |
| 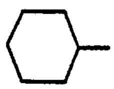 $CH_2$– | H |

Example 4

12-Allyl-5,6-dihydro-11-methylbenzo[5,6]cyclohepta[1,2-c]pyridin-6,11-imine

A mixture of 2.45 g. of 5,6-dihydro-11-methylbenzo[5,6]cyclohepta[1,2-c]pyridin-6,11-imine, 1.8 g. of allyl bromide, 3.0 g. of anhydrous sodium carbonate and 50 ml. of dry toluene is heated at 80°C. for 20 hours. The mixture is filtered and the filtrate is evaporated *in vacuo* to give 12-allyl-5,6-dihydro-11-methylbenzo[5,6]cyclohepta[1,2-c]pyridin-6,11-imine which is converted to its hydrogen fumarate salt.

Employing the procedure substantially as described in Example 4, but using as starting materials the benzo[5,6]cyclohepta[1,2-c]pyridin-6,11-imines and the R-halides described in Table IV, there are produced the 12-R-benzo-[5,6]-cyclohepta[1,2-c]pyridin-6,11-imines also described in Table IV in accordance with the following reaction:

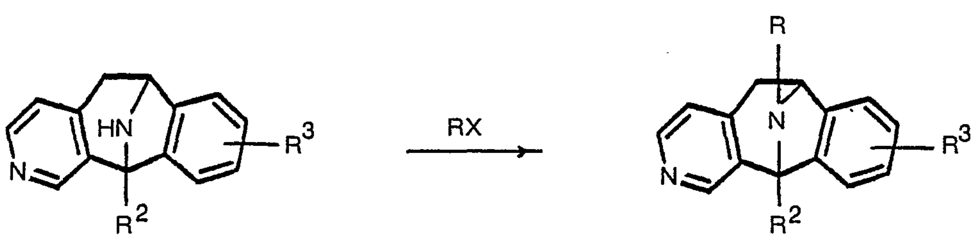

TABLE IV

| R | X | $R^2$ | $R^3$ |
|---|---|---|---|
| 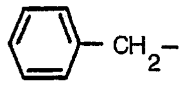 | Cl | $-CH_3$ | H |
| $C_2H_5-$ | Br | $-CH_3$ | H |
| $CH_2=CHCH_2-$ | Br | $-CH_3$ | 9-Br |
| $C_2H_5-$ | Br | $-C_2H_5$ | H |
| $C_2H_5-$ | Br | $-CH_3$ | 9-F |
| 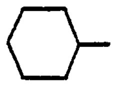 | Br | $-CH_3$ | H |
| 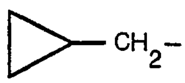 | Br | $-CH_3$ | H |
| $(CH_3)_2NCH_2CH_2CH_2-$ | Br | $-CH_3$ | H |
| $CH_3-$ | I | $-CH_3$ | 9-Br |
| $CH_3-$ | I | $-CH_3$ | H |

Example 5

12-Benzyl-5,6-dihydro-11-methylbenzo[5,6]cyclohepta[1,2-c]pyridin-6,11-imine

To a solution of 5,6-dihydro-11-methylbenzo[5,6]cyclohepta[1,2-c]pyridin-6,11-imine (2.35 g.) and benzaldehyde (1.1 g.) in THF (100 ml.) is added acetic acid (1 ml.) and sodium cyanoborohydride (1.0 g.). The mixture is stirred for two days, filtered and the filtrate evaporated. The residue is slurried with 1N aqueous $NH_4OH$ and extracted with $HCCl_3$. The chloroform extract is dried over sodium sulfate, filtered and evaporated. The residue is recrystallized from ethanol to yield 12-benzyl-5,6-dihydro-11-methylbenzo[5,6]cyclohepta[1,2-c]pyridin-6,11-imine.

Employing the procedure substantially as described in Example 5 but using as starting materials the benzo[5,6]cyclohepta[1,2-c]pyridin-6,11-imines and the aldehydes described in Table V there are produced the 12-R-benzo[5,6]cyclohepta[1,2-c]pyridin-6,11-imines also described in Table V in accordance with the following reaction:

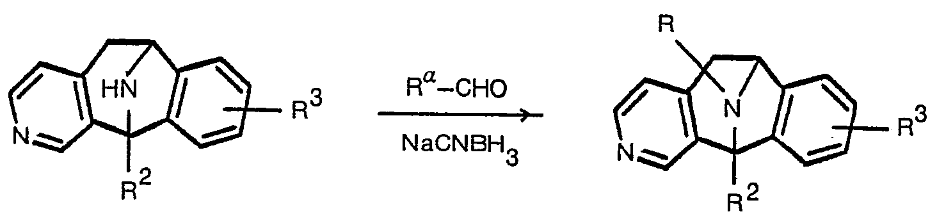


TABLE V

| $R^\alpha$ | R | $R^2$ | $R^3$ |
|---|---|---|---|
| $CH_3-$ | $-C_2H_5$ | $-CH_3$ | H |
| $CH_3-$ | $-C_2H_5$ | $-CH_3$ | 9-Br |
| $CH_3$ | $-C_2H_5$ | $-CH_3$ | 9-F |

Example 6

5,6-Dihydro-12-ethyl-11-methylbenzo[5,6]cyclohepta[1,2-c]pyridin-6,11-imine

An ice cold solution of 5,6-dihydro-11-methylbenzo[5,6]cyclohepta[1,2-c]pyridin-6,11-imine (2.35 g.) and triethylamine (2.0 g.) in ether (100 ml.) is treated dropwise with acetyl chloride (1.5 g.). After 10 hours, the solution is washed with water, dried over sodium sulfate, filtered, and the filtrate evaporated to dryness. The residue is redissolved in ether (200 ml.) and 400 mg. of $LiAlH_4$ added. The resulting slurry is stirred for 24 hours. Water is added slowly and the resulting slurry filtered. The filtrate is dried over sodium sulfate, filtered and the filtrate evaporated to yield 5,6-dihydro-12-ethyl-11-methylbenzo[5,6]cyclohepta[1,2-c]pyridin-6,11-imine.

Employing the procedure substantially as described in Example 6, but using as starting materials the benzo[5,6]cyclohepta[1,2-c]pyridin-6,11-imines and the alkanoyl halides described in Table VI there are produced the 12-R-benzo[5,6]cyclohepta[1,2-c]pyridin-6,11-imines also described in Table VI in accordance with the following reaction:

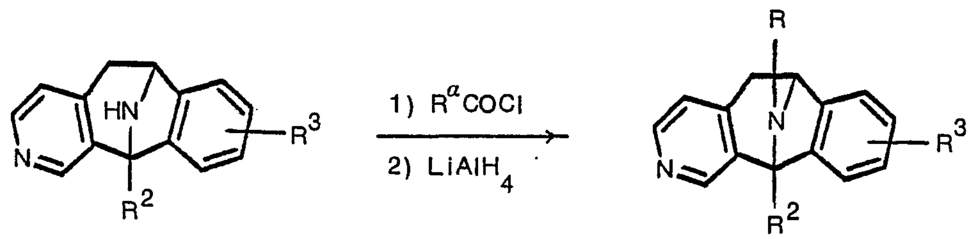


TABLE VI

| $R^{\alpha}$ | R | $R^2$ | $R^3$ |
|---|---|---|---|
| $C_2H_5O$— | —$CH_3$ | —$CH_3$ | H |
| $C_2H_5O$— | —$CH_3$ | —$CH_3$ | 9-Br |
| 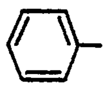 | -$CH_2$- 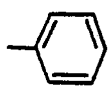 | —$CH_3$ | 9-F |

Example 7

5,6-Dihydro-6,11-dimethyl-12-phenylbenzo[5,6]cyclohepta[1,2-c]pyridin-6,11-imine

A mixture of aniline (1.96 ml.), trifluoroacetic acid (5 ml.) and 6,11-dimethylbenzo[5,6]cyclohepta[1,2-c]pyridin-11-ol is stirred at ambient temperature for 18 hours. It is then heated to reflux with an oil bath for one hour and neutralized.

Gas liquid chromatography indicates 11 mol% starting material, 53 mol% 6-methylbenzo[5,6]-cyclohepta(1,2-c)pyridin-11-methylene, and 33 mol% 5,6-dihydro-6,11-dimethyl-12-phenyl-benzo[5,6]cyclohepta[1,2-c]pyridin-6,11-imine. The title compound is isolated by chromatography on silica gel with equal parts of chloroform and ethyl acetate, and is recrystallized from cyclohexane, m.p. 165—185°C.

Anal. Calc. for $C_{22}H_{20}N_2$ (312.40): C, 84.58; H, 6.45; N, 8.97.
Found: C, 83.70; H, 6.41; N, 8.94.

Example 8

10,11-Dihydro-12-methyl-5H-benzo[4,5]cyclohepta[1,2-b]pyridin-5,10-imine

*Step A:*

Preparation of 5-methylamino-5H-benzo[4,5]cyclohepta[1,2-b]pyridine

Methylamine (3.72 g., 0.12 mole) is added to a mechanically stirred mixture of benzo[4,5]cyclohepta[1,2-b]pyridin-5-one (6.0 g., 0.03 mole) in benzene (150 ml.). A solution of $TiCl_4$ (2.1 ml.) in benzene (50 ml.) is added and the mixture is stirred at 25°C. for 18 hours. Anhydrous potassium carbonate is added to the mixture and it is filtered through diatomaceous earth. The filtrate is evaporated to an amber oil (5.47 g.) which is dissolved in acetonitrile (150 ml.). Sodium borohydride (5.47 g., 0.025 mole) is added and the mixture is heated on a steam bath for 1 1/2 hours. Dilute HCl (3*N*) is added to the cooled reaction mixture and it is evaporated to dryness under reduced pressure. The residue is dissolved in 3*N* HCl (150 ml.), extracted with ether and the aqueous filtrate is made alkaline by the addition of 40% weight per volume NaOH solution. The basic material is extracted into

$CHCl_3$ and concentrated to an amber oil (3.96 g.). This oil is converted to a hydrochloride salt by treatment with ethanolic hydrogen chloride for use in Step B.

*Step B:*
Preparation of 10,11-dihydro-12-methyl-5H-benzo[4,5]cyclohepta[1,2-b]pyridin-5,10-imine

An ethanol solution of the hydrochloride salt of 5-methylamino-5H-benzo[4,5]cyclohepta[1,2-b]-pyridine from Step A is heated in an oil bath (90°C.) for three days. The cooled solution is evaporated to dryness and the resultant foam is dissolved in water (50 ml.). The aqueous solution is extracted with ether and then made alkaline by the addition of 20% weight per volume NaOH solution. The basic material is extracted into $CHCl_3$ and concentrated to an amber oil (2.90 g.). Silica gel chromatography provides the product as a yellow crystalline solid (1.33 g.). Recrystallization from hexane gives 10,11-dihydro-12-methyl-5H-benzo[4,5]cyclohepta[1,2-b]pyridin-5,10-imine, m.p. 113°—115°C.

Employing the procedure substantially as described in Example 8, Steps A and B but substituting for the methylamine used in Step A, an equimolecular amount of an amine of formula R—$NH_2$, wherein R is

$-C_3H_7$-n, $-CH_2-CH=CH_2$, $-CH_2$ 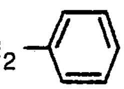 , $-CH_2$ 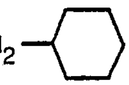 , 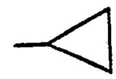

or $—CH_2CH_2CH_2N(CH_3)_2$ there is produced respectively a 10,11-dihydro-12-R-benzo[4,5]cyclohepta[1,2-b]pyridin-5,10-imine, of formula

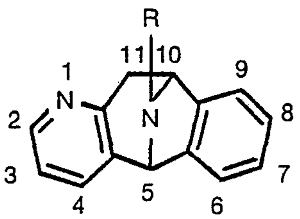

wherein R is as defined immediately above.

Example 9

5,6-Dihydro-12-methyl-11H-benzo[5,6]cyclohepta[1,2-b]pyridin-5,11-imine

*Step A:*
Preparation of 5,6-dibromo-5,6-dihydrobenzo[5,6]cyclohepta[1,2-b]pyridin-11-one

To a solution of benzo[5,6]cyclohepta[1,2-b]pyridin-11-one (5.20 g., 0.025 mole) in chloroform (100 ml.) is added pyridine perbromide (12.0 g. 0.050 mole). The resultant solution is allowed to stir at 25°C. for 24 hours, and then is heated on the steam bath for 24 hours. Evaporation to dryness produces an amber residue. This residue is taken up in water (100 ml.), made alkaline with saturated sodium carbonate solution, and the basic material is extracted into $CHCl_3$. The $CHCl_3$ is removed *in vacuo*, and the residual oil is triturated with acetone (20 ml.) to yield the product as a brown solid, m.p. 148°—158°C. (dec.).

*Step B:*
Preparation of 6$\beta$-bromo-5,6-dihydro-11$\alpha$-hydroxy-12-methyl-11H-benzo[5,6]cyclohepta[1,2-b]pyridin-5,11-imine

To a solution of 5,6-dibromo-5,6-dihydrobenzo[5,6]cyclohepta[1,2-b]pyridin-11-one (1.0 g., 0.00273 mole) in tetrahydrofuran (100 ml.) is added a solution of $CH_3NH_2$ (0.13 g., 0.0041 mole) in tetrahydrofuran (25 ml.). The resulting amber solution is stirred at 25°C. for 24 hours. Methylamine hydrobromide is filtered off and the filtrate is evaporated to dryness to yield the product as a tan solid (0.75 g.). Recrystallization from benzene gives 6-$\beta$-bromo-5,6-dihydro-11$\alpha$-hydroxy-12-methyl-11H-benzo[5,6]cyclohepta[1,2-b]pyridin-5,11-imine, m.p. 143°—145°C. dec.

*Step C:*
Preparation of 6$\beta$-bromo-11$\alpha$-chloro-5,6-dihydro-12-methyl-11H-benzo[5,6]cyclohepta[1,2-b]pyridin-5,11-imine

Thionyl chloride (20 ml.) is added to a cooled solution of 6$\beta$-bromo-5,6-dihydro-11$\alpha$-hydroxy-12-methyl-11H-benzo[5,6]cyclohepta[1,2-b]pyridin-5,11-imine (2 g.) in toluene (50 ml.). The temperature of the resulting mixture is raised to ambient temperature and warmed on the steam bath for 0.5 hours. Anhydrous potassium carbonate is added and the mixture filtered. The filtrate is evaporated under reduced pressure to give 6$\beta$-bromo-11$\alpha$-chloro-5,6-dihydro-12-methyl-11H-benzo[5,6]cyclohepta[1,2-b]pyridin-5,11-imine.

*Step D:*
Preparation of 5,6-dihydro-12-methyl-11H-benzo[5,6]cyclohepta[1,2-b]pyridin-5,11-imine

The oil obtained in Step C is dissolved in 50 ml. of ethyl acetate, triethylamine (5 ml.) and 0.20 g. of 10 weight/weight% palladium on carbon is added and the mixture is hydrogenated under 2.81—3.52 kg/cm$^2$ (40—50 lbs/in$^2$) of hydrogen pressure. The catalyst and triethylammonium salts are removed by filtration and the filtrate is evaporated under reduced pressure. The oily residue is chromatographed over silica gel using chloroform as the eluent to give 5,6-dihydro-12-methyl-11H-benzo(5,6)cyclohepta(1,2-b) pyridin-5,11-imine.

Employing the procedure substantially as described in Example 9, but substituting for the methylamine used in Step B thereof, an equimolecular amount of an amine of formula R—$NH_2$, wherein R is

$-C_3H_2$-n, $CH_2$ $-CH=CH_2$, $-CH_2$ 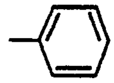 , $-CH_2$ 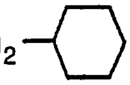 , 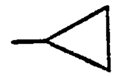 ,

or —$CH_2CH_2N(CH_3)_2$, there is produced the 5,6-dihydro-12-R-11H-benzo[5,6]cyclohepta[1,2-b]-pyridin-5,11-imine in accordance with the following reaction scheme:

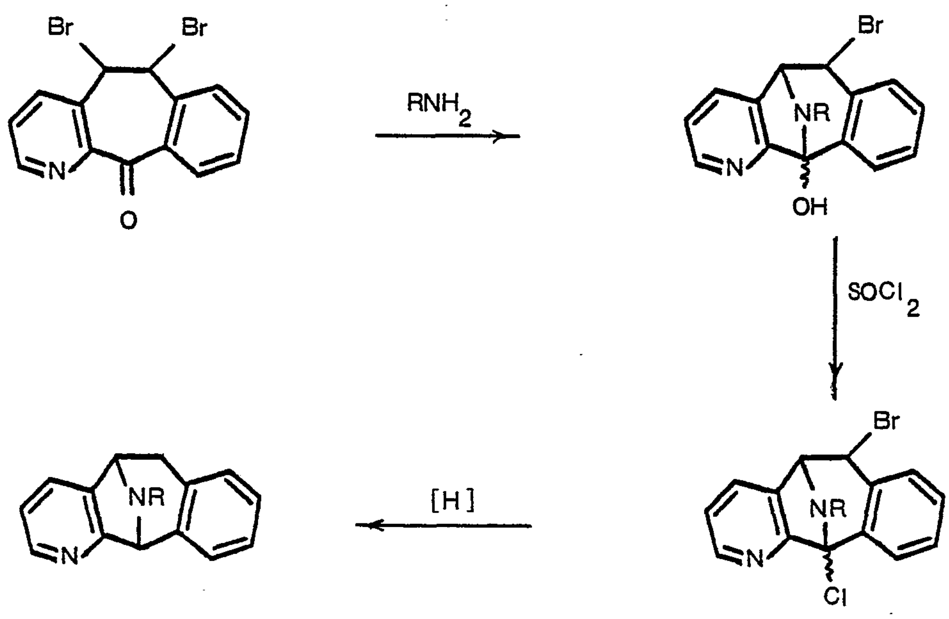


wherein R is as defined immediately above.

Example 10
5,6-Dihydro-11-methylbenzo[5,6]cyclohepta [1,2-c]pyridin-6,11-imine

*Step A:*
Preparation of 11-imino-benzo[5,6]cyclohepta[1,2-c]pyridine

Ammonia (gas) is bubbled into a chilled, stirred mixture of benzo[5,6]cyclohepta[1,2-c]pyridin-11-one (10 g., 0.048 mol), titanium tetrachloride (3.5 ml) and toluene (1000 ml) for 0.25 hours. The bath is removed and the resulting mixture is stirred at ambient temperature for four hours. Saturated sodium carbonate solution is added (1000 ml) and the mixture filtered to remove titanium salts. The layers are separated, the aqueous phase is extracted with ethyl acetate and the combined organic layers are washed with water, saturated aqueous sodium chloride solution, and dried over anhydrous sodium sulfate. The solvent is removed *in vacuo*, 6.5 g of 11-iminobenzo[5,6]cyclohepta[1,2-c]pyridine are obtained.

*Step B:*
Preparation of 11-(p-toluenesulfonimino)benzo[5,6]cyclophepta[1,2-c]pyridine

p-Toluenesulfonyl chloride (2.68 g, 0.014 mol) is added to a chilled stirred mixture of 11-imino-benzo[5,6]cyclohepta[1,2-c]pyridine (2.6 g, 0.013 mol) and the cooling bath is removed. After 3 hours at 25°, the pyridine is removed *in vacuo* and the residue is recrystallized from EtOH to give 3.2 g, of 11-(p-toluenesulfonimino)benzo[5,6]cyclohepta[1,2-c]pyridine.

*Step C:*
Preparation of 11-methyl-11-(p-toluenesulfonamido)benzo[5,6]cyclohepta[1,2-c]pyridine

Methyllithium (15 ml, 1.4M in ether) is added to 11-(p-toluenesulfonimino)benzo[5,6]cyclohepta[1,2-c]pyridine (3.6 g, 0.01 mol) in tetrahydrofuran (25 ml.). After two hours, the reaction mixture is diluted with 10% weight per volume ammoniumchloride solution and extracted with ethyl acetate. The extract is washed with water, saturated aqueous sodium chloride solution and dried over anhydrous sodium sulfate. Removal of the dried solvent *in vacuo* gives 3.0 g of 11-methyl-11-(p-toluenesulfonamido)benzo[5,6]cyclohepta[1,2-c]pyridine.

*Step D:*
Preparation of 5,6-dihydro-11-methyl-12-(p-toluenesulfonyl)benzo[5,6]cyclohepta[1,2-c]-pyridin-6,11-imine

A mixture of 11-methyl-11-(p-toluenesulfonamido)benzo[5,6]cyclohepta[1,2-c]pyridine (1.2 g), sodium hydroxide (2.4 g), water (24 ml) and dioxane (24 ml) is heated to reflux for 2 hours. The reaction mixture is cooled, diluted with water and extracted with ethyl acetate. The extracts are washed with water, saturated sodium chloride solution, and dried over anhydrous sodium sulfate. Removal of the solvent *in vacuo* produces 1.0 g of 5,6-dihydro-11-methyl-12-(p-toluenesulfonyl)benzo[5,6]cyclohepta[1,2-c]pyridin-6,11-imine.

*Step E:*
Preparation of 5,6-dihydro-11-methylbenzo[5,6]cyclohepta[1,2-c]pyridin-6,11-imine fumarate

A solution of 5,6-dihydro-11-methyl-12-(p-toluenesulfonyl)benzo[5,6]cyclohepta[1,2-c]pyridine-6,11-imine (1.0 g) in acetic acid (10 ml) and concentrated hydrochloric acid (5 ml) is heated to reflux for 24 hours. The cooled reaction mixture is diluted with (150 ml), made alkaline by the addition of 20% sodium hydroxide solution, and extracted with ethyl acetate. The extracts are washed with water, saturated sodium chloride solution, and dried over anhydrous sodium sulfate. Removal of the solvent *in vacuo* and treatment of the residue with a boiling solution of fumaric acid in acetone gives 0.72 g of 5,6-dihydro-11-methylbenzo[5,6]cyclohepta[1,2-c]pyridin-6,11-imine fumarate.

*Alternative Step E:*

A solution of 5,6-dihydro-11-methyl-12-(p-toluenesulfonyl)benzo[5,6]cyclohepta[1,2-c]pyridin-6,11-imine (100 mg.) and 1 g. of a 70% (w/w) solution in benzene of sodium bis(2-methoxyethoxy)aluminum hydride in 15 ml. of toluene is stirred at room temperature for 24 hours. The mixture is poured into dilute caustic solution (2% w/v NaOH) and extracted with ethyl acetate. The ethyl acetate is concentrated to dryness to give 5,6-dihydro-11-methylbenzo[5,6]cyclohepta[1,2-c]pyridin-6,11-imine which is converted to the fumarate.

Following the procedure substantially as described in Example 10, but substituting for the benzo[5,6]cyclohepta[1,2-c]pyridin-11-one used in Step A, and the methyllithium used in Step C, the 6-$R^1$-9-$R^3$-benzo[5,6]cyclohepta[1,2-c]pyridin-11-ones described in Table VII there are produced the 6-$R^1$-9-$R^3$-11-$R^2$-5,6-dihydro-benzo[5,6]cyclohepta[1,2-c]pyridin-6,11-imines also described in Table VII by the following process:

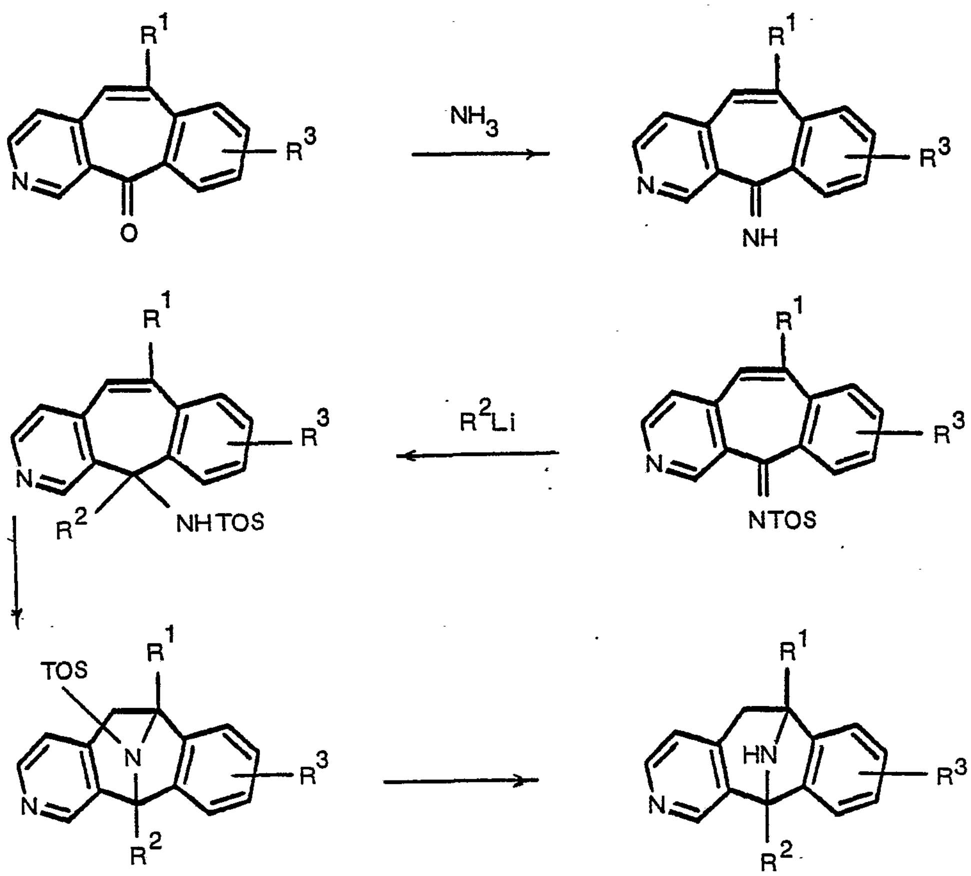


TABLE VII

| $R^1$ | $R^2$ | $R^3$ |
|---|---|---|
| $CH_3$ | $CH_3$ | 9-Br |
| $CH_3$ | $CH_3$ | 9-F |
| $CH_3$ | $CH_3$ | H |
| $CH_3$ | $C_2H_5$ | H |

Example 11

*Preparation of intravenous solutions*

A solution containing 10 mg. of 5,6-dihydro-11-methylbenzo[5,6]cyclohepta[1,2-c]pyridin-6,11-imine hydrogen fumarate per ml. of injectable solution is prepared in the following manner.

A mixture of 10 mg. of active ingredient and 9 mg. of sodium chloride is dissolved in sufficient water for injection to make 1 ml. of solution. The pH is adjusted using hydrochloric acid or aqueous sodium hydroxide to about pH 7.0.

If it is desired that the intravenous solution be used for multi-dose purposes, 1.0 mg of methyl-p-hydroxy-benzoate (methyl paraben) and 0.10 mg. of n-propyl-p-hydroxy benzoate (propyl paraben) are mixed with the other solids before adding water to dissolve the solids. The solution is prepared and stored in such a manner that it is suitably protected from the deleterious effects of the atmosphere. One method by which this can be accomplished is by preparation and storage of the solution in an atmosphere of nitrogen. The resulting solution is sterilized by autoclaving. Injectable solutions comprising 0.1, 1.0, 100.0 mg., respectively, of active ingredient per ml. of solution are similarly prepared substituting the indicated amount for the above-illustrated 10 mg. of quantity. Bulk injectable solutions of convenient volume for subsequent delivery in unit dosage form are readily prepared following the above procedure.

Following the above procedure, other representative injectable solutions of the present invention are prepared when the active ingredient used in 11 is replaced by an equivalent amount of any of the novel compounds of the present invention.

Example 12

*Tablet Preparation*

Tablets containing 1.0, 2.0, 25.0, respectively, of 5,6-dihydro-11-methylbenzo[5,6]cyclohepta-[1,2-c]pyridin-6,11-imine are prepared as illustrated below.

TABLE FOR DOSES CONTAINING FROM
1—25 MG. OF THE ACTIVE COMPOUND

| | Amount—mg. | | |
|---|---|---|---|
| 5,6-dihydro-11-methylbenzo[5,6]-cyclohepta[1,2-c]pyridin-6,11-imine | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

All of the active compound, cellulose, and a portion of the corn starch are mixed and granulated to a 10% weight per weight corn starch paste. The resulting granulate is sieved, dried and blended with the remainder of the corn starch and the magnesium stearate. The resulting granulate is then compressed into tablets containing 1.0 mg., 2.0 mg., 25.0 mg., of active ingredient per tablet. Other tablets are prepared using the same procedures and the equivalent amounts of excipients along with equivalent amounts of any of the novel compounds of the present invention.

**Claims**

1. A compound of structural formula:

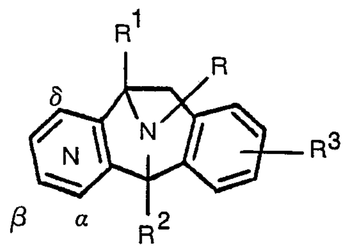

or

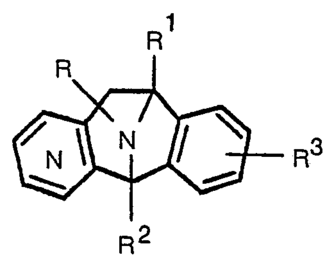

or a pharmaceutically acceptable salt thereof, wherein
$R^1$ and $R^2$ are independently
(1) hydrogen
(2) an alkyl group having 1—5 carbon atoms,
(3) an alkenyl group having 2—5 carbon atoms,
(4) a phenylalkyl group having 1—3 carbon atoms in the alkyl moiety,
(5) a cycloalkyl group having 3—6 carbon atoms,
(6) a cycloalkylalkyl group having 3—6 carbon atoms in the cycloalkyl moiety, and 1—3 carbon atoms in the alkyl moiety,
R is
(1) $R^1$,
(2) phenyl, or
(3) di($C_{1-3}$alkyl)amino-$C_{1-5}$alkyl, and
$R^3$ is
(1) hydrogen,
(2) halogen,
(3) an alkoxy group having 1—5 carbon atoms,
(4) trifluoromethylthio,
(5) cyano, or
(6) carboxy.

2. The compound of claim 1 or a pharmaceutically acceptable salt thereof, wherein
$R^1$ and $R^2$ are independently
(1) hydrogen,

(2) methyl or ethyl,
(3) vinyl or allyl,
(4) benzyl,
(5) cyclopropyl or cyclohexyl,
(6) a cycloalkylalkyl group having 3—6 carbon atoms in the cycloalkyl moiety, and 1—3 carbon atoms in the alkyl moiety;

R is
(1) $R^1$,
(2) phenyl, or
(3) di($C_{1-3}$alkyl)amino-$C_{1-5}$alkyl, and

$R^3$ is
(1) hydrogen,
(2) chloro, bromo, fluoro or iodo,
(3) methoxy,
(4) trifluoromethylthio,
(5) cyano, or
(6) carboxy.

3. The compound of claim 1 or 2, wherein N of the pyridine ring is in the $\alpha$, $\beta$, or $\delta$ position.

4. The compound of claim 3, wherein $R^3$ is hydrogen, $R^1$ is hydrogen, and R and $R^2$ are independently hydrogen, alkyl having 1—5 carbon atoms or benzyl.

5. The compound of claim 4 of structural formula:

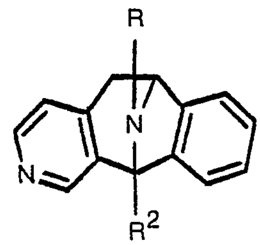

wherein
R is hydrogen, alkyl having 1—5 carbon atoms or benzyl, and
$R^2$ is hydrogen or alkyl having 1—5 carbon atoms.

6. A process for the preparation of a compound according to claim 1 which comprises: treating a compound of structural formula:

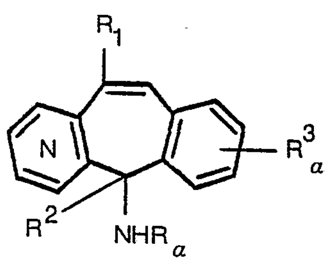

or

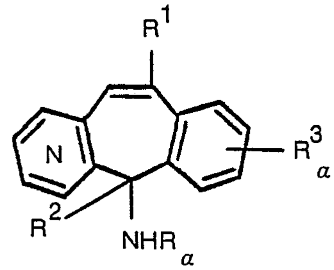

wherein
$R_\alpha$ is R, lower alkanoyl, phenyl-sulfonyl, tosyl, alkoxycarbonyl, diphenylphosphinyl or arylsulfonyl, and
$R^3_\alpha$ is hydrogen or halogen,
with acid to produce the compound of structural formula:

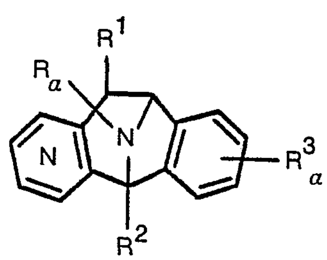

or

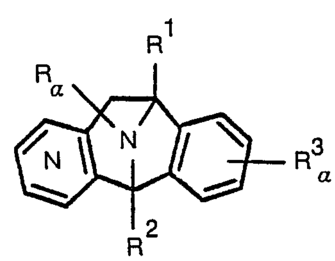

followed by hydrolysis or hydrogenolysis of $R_\alpha$, where $R_\alpha$ is other than R, and which optionally comprises a further treatment of the compounds, in which $R^3_\alpha$ is bromo, with either

(a) a corresponding alkali metal alkoxide to produce the compound wherein $R^3$ is alkoxy having 1—5 carbon atoms, or

(b) trifluoromethylthio-copper to produce the compound wherein $R^3$ is trifluoromethyl, or

(c) cuprous cyanide to produce the compound wherein $R^3$ is cyano, and which optionally comprises

(d) a following hydrolysis of the compound resulting from step (c), in which $R^3$ is cyano to produce the compound wherein $R^3$ is carboxy.

7. A process for the preparation of a compound of structural formula:

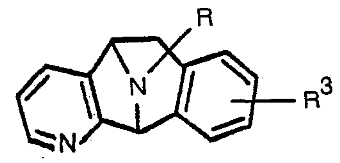

wherein
R is

(1) hydrogen
(2) an alkyl group having 1—5 carbon atoms,
(3) an alkenyl group having 2—5 carbon atoms,
(4) a phenylalkyl group having 1—3 carbon atoms in the alkyl moiety,
(5) a cycloalkyl group having 3—6 carbon atoms,
(6) a cycloalkylalkyl group having 3—6 carbon atoms in the cycloalkyl moiety, and 1—3 carbon atoms in the alkyl moiety, or
(7) di($C_{1-3}$alkyl)amino-$C_{1-5}$alkyl, and

$R^3$ is as defined in claim 1, which comprises hydrogenolysis of the X-groups in a compound of formula:

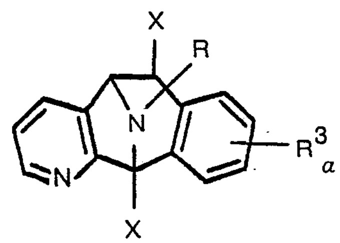

wherein
X is Br or Cl, and
$R^3_\alpha$ is hydrogen or halogen,
to produce a compound of formula

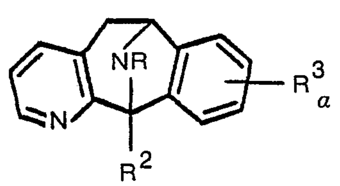

and comprises optionally a further treatment of the compound, wherein $R^3_\alpha$ is bromo, with either

(a) either a corresponding alkali metal alkoxide to produce the compound wherein $R^3$ is an alkoxy group having 1—5 carbon atoms, or with

(b) trifluoromethylthio-copper to produce the compound wherein $R^3$ is trifluoromethyl, or

(c) cuprous cyanide to produce the compound wherein $R^3$ is cyano, and comprises optionally

(d) a further hydrolysis of the compounds of step (c), wherein $R^3$ is cyano to produce the compound wherein $R^3$ is carboxy.

8. A process for the preparation of a compound of structural formula:

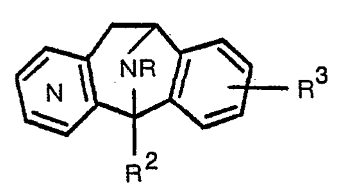

wherein R, $R^2$ and $R^3$ are as defined in claim 1, which comprises

(a) a reduction of a compound of formula

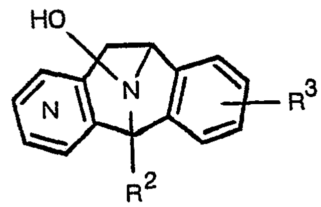

to produce the compound wherein R is hydrogen, followed optionally by

(b) an introduction of the radical R in the meaning of an alkyl group having 1—5 carbon atoms, an alkenyl group having 2—5 carbon atoms, a phenylalkyl group having 1—3 carbon atoms in the alkyl moiety, a cycloalkyl group having 3—6 carbon atoms, a cycloalkylalkyl group having 3—6 carbon atoms in the cycloalkyl moiety and 1—3 carbon atoms in the alkyl moiety, a phenyl group or a di($C_{1-3}$alkyl)amino-$C_{1-5}$alkyl into the compounds of formula:

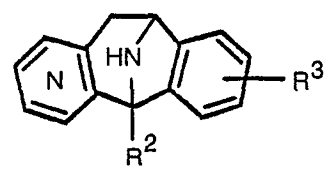

according to the treatment of the N-alkylation to produce the compound wherein R has the above outlined meaning and/or optionally

(c) a treatment of the compound wherein $R^3$ is bromo with a corresponding sodium or potassium alkoxide, with cuprous cyanide or with bis(trifluoromethylthio) mercury and copper dust to produce the compounds wherein $R^3$ is alkoxy having 1—5 carbon atoms, cyano or trifluoromethylthio respectively; and optionally compirising

(d) a following hydrolysis of the compound resulting from step (c), wherein $R^3$ is cyano, to produce the compound wherein $R^3$ is carboxy.

9. The process of claim 8, wherein the N of the pyridine ring is in the $\beta$-position.

10. A pharmaceutical composition comprising a pharmaceutical carrier and an effective amount of a compound according to claim 1, or a pharmaceutically acceptable salt thereof.

**Patentansprüche**

1. Verbindung mit der Strukturformel

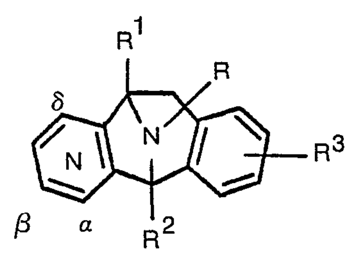

oder

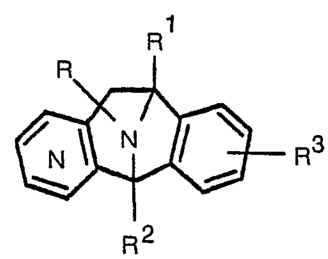

oder ein pharmazeutisch brauchbares Salz davon, worin
$R^1$ und $R^2$ unabhängig sind
(1) Wasserstoff,
(2) eine Alkylgruppe mit 1—5 Kohlenstoffatomen,
(3) eine Alkenylgruppe mit 2—5 Kohlenstoffatomen,
(4) eine Phenylalkylgruppe mit 1—3 Kohlenstoffatomen im Alkylrest,
(5) eine Cycloalkylgruppe mit 3—6 Kohlenstoffatomen,
(6) eine Cycloalkylalkylgruppe mit 3—6 Kohlenstoffatomen im Cycloalkylrest und 1—3 Kohlenstoffatomen im Alkylrest,
R die Bedeutung hat von
(1) $R^1$,
(2) Phenyl oder
(3) Di-($C_{1-3}$-alkyl)-amino-$C_{1-5}$-alkyl, und
$R^3$ die Bedeutung hat von
(1) Wasserstoff,

(2) Halogen,
(3) einer Alkoxygruppe mit 1—5 Kohlenstoffatomen,
(4) Trifluormethylthio,
(5) Cyano oder
(6) Carboxy.

2. Verbindung nach Anspruch 1 oder ein pharmazeutisch brauchbares Salz davon, worin $R^1$ und $R^2$ unabhängig bedeuten
(1) Wasserstoff,
(2) Methyl oder Ethyl,
(3) Vinyl oder Allyl,
(4) Benzyl,
(5) Cyclopropyl oder Cyclohexyl,
(6) eine Cycloalkylalkylgruppe mit 3—6 Kohlenstoffatomen im Cycloalkylrest und 1—3 Kohlenstoffatomen im Alkylrest;

R die Bedeutung hat von
(1) $R^1$,
(2) Phenyl oder
(3) Di-($C_{1-3}$-alkyl)-amino-$C_{1-5}$-alkyl und

$R^3$ die Bedeutung hat von
(1) Wasserstoff,
(2) Chlor, Brom, Fluor oder Jod,
(3) Methoxy,
(4) Trifluormethylthio,
(5) Cyano oder
(6) Carboxy.

3. Verbindung nach Anspruch 1 oder 2, worin das N des Pyridinrings in der $\alpha$, $\beta$ oder $\delta$-Stellung ist.

4. Verbindung nach Anspruch 3, worin $R^3$ Wasserstoff ist, $R^1$ Wasserstoff ist und R und $R^2$ unabhängig Wasserstoff, Alkyl mit 1—5 Kohlenstoffatomen oder Benzyl sind.

5. Verbindung nach Anspruch 4 mit der Strukturformel

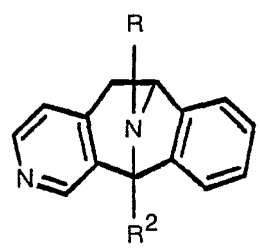

worin
R Wasserstoff, Alkyl mit 1—5 Kohlenstoffatomen oder Benzyl ist und
$R^2$ Wasserstoff oder Alkyl mit 1—5 Kohlenstoffatomen ist.

6. Verfahren zur Herstellung einer Verbindung nach Anspruch 1, durch Behandeln einer Verbindung der Strukturformel

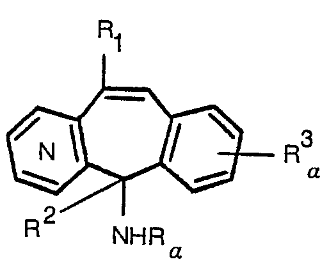

oder

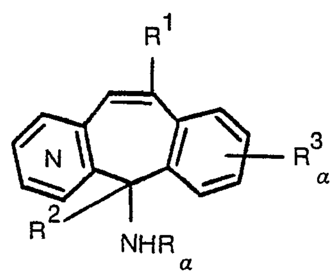

worin
$R_\alpha$ die Bedeutung hat von R, niedrig-Alkanoyl, Phenylsulfonyl, Tosyl, Alkoxycarbonyl, Diphenylphosphinyl oder Arylsulfonyl, und
$R_\alpha^3$ Wasserstoff oder Halogen ist,
mit einer Säure, unter Bildung der Verbindung der Strukturformel

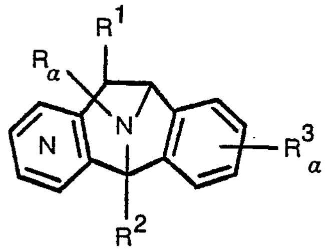

oder

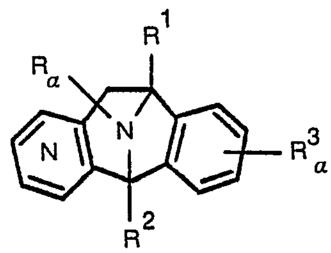

gefolgt von der Hydrolyse oder Hydrogenolyse von $R_\alpha$, worin $R_\alpha$ unterschiedlich von R ist, und, das gegebenenfalls eine weitere Behandlung der Verbindungen umfaßt, worin $R^3_\alpha$ Brom ist, mit entweder

(a) einem entsprechenden Alkalimetallalkoxid, unter Bildung der Verbindung, worin $R^3$ Alkoxy mit 1—5 Kohlenstoffatomen ist oder

(b) Trifluormethylthio-Kupfer, unter Bildung einer Verbindung, worin $R^3$ Trifluormethyl ist, oder

(c) Kupfer-I-cyanid, unter Bildung der Verbindung, worin $R^3$ Cyano ist und das gegebenenfalls umfaßt

(d) eine folgende Hydrolyse der aus der Stufe (c) resultierenden Verbindung, worin $R^3$ Cyano ist, unter Bildung der Verbindung, worin $R^3$ Carboxy ist.

7. Verfahren zur Herstellung einer Verbindung der Strukturformel

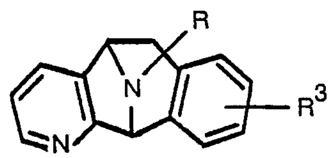

worin

R die Bedeutung hat von

(1) Wasserstoff,

(2) einer Alkylgruppe mit 1—5 Kohlenstoffatomen,

(3) einer Alkenylgruppe mit 2—5 Kohlenstoffatomen,

(4) einer Phenylalkylgruppe mit 1—3 Kohlenstoffatomen im Alkylrest,

(5) einer Cycloalkylgruppe mit 3—6 Kohlenstoffatomen,

(6) einer Cycloalkylalkylgruppe mit 3—6 Kohlenstoffatomen im Cycloalkylrest und 1—3 Kohlenstoffatomen im Alkylrest oder

(7) Di-($C_{1-3}$-alkyl)-amino-$C_{1-5}$-alkyl, und

$R^3$ wie in Anspruch definiert ist,

durch Hydrogenolyse der X-Gruppen in einer Verbindung der Formel

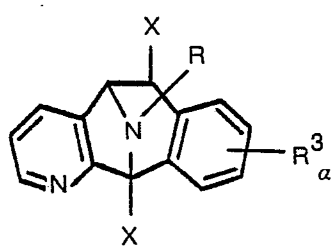

worin

X Br oder Cl ist und

$R^3_\alpha$ Wasserstoff oder Halogen ist,

unter Bildung einer Verbindung der Formel

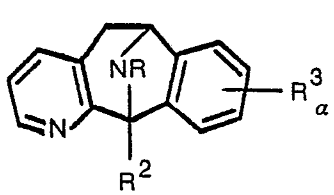

und das gegebenenfalls eine weitere Behandlung der Verbindung umfaßt, worin $R^3_\alpha$ Brom ist, mit entweder

(a) einem entsprechenden Alkalimetallalkoxid, unter Bildung der Verbindung, worin $R^3$ eine Alkoxygruppe mit 1—5 Kohlenstoffatomen ist, oder mit

(b) Trifluormethylthio-Kupfer, unter Bildung der Verbindung, worin $R^3$ Trifluormethyl ist, oder

(c) Kupfer-I-cyanid, unter Bildung der Verbindung, worin $R^3$ Cyano ist, das gegebenenfalls umfaßt

(d) eine weitere Hydrolyse der Verbindungen der Stufe (c), worin $R^3$ Cyano ist, unter Bildung der Verbindung, worin $R^3$ Carboxy ist.

8. Verfahren zur Herstellung einer Verbindung der Strukturformel

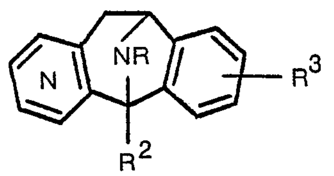

worin R, $R^2$ und $R^3$ wie in Anspruch 1 definiert sind durch

(a) Reduktion einer Verbindung der Formel

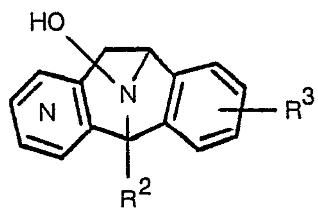

unter Bildung einer Verbindung, worin R Wasserstoff ist, gefolgt gegebenenfalls von

(b) Einführen des Rests R in der Bedeutung einer Alkylgruppe mit 1—5 Kohlenstoffatomen, einer Alkenylgruppe mit 2—5 Kohlenstoffatomen, einer Phenylalkylgruppe mit 1—3 Kohlenstoffatomen im Alkylrest, einer Cycloalkylgruppe mit 3—6 Kohlenstoffatomen, einer Cycloalkylalkylgruppe mit 3—6 Kohlenstoffatomen im Cycloalkylrest und 1—3 Kohlenstoffatomen im Alkylrest, einer Phenylgruppe oder eines Di-($C_{1-3}$-alkyl)-amino-$C_{1-5}$-alkyl in die Verbindung der Formel

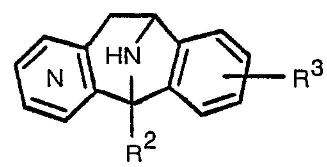

gemäß einer N-alkylierungsbehandlung, unter Bildung der Verbindung, worin R die vorstehend umrissene Bedeutung aufweist und/oder gegebenenfalls.

(c) einer Behandlung der Verbindung, worin $R^3$ Brom ist mit einem entsprechenden Natrium- oder Kaliumalkoxid, mit Kupfer-I-cyanid oder mit Bis-(trifluormethylthio)-Quecksilber und Kupferstaud, unter Bildung der Verbindungen, worin $R^3$ Alkoxy mit 1—5 Kohlenstoffatomen, Cyano bzw. Trifluormethylthio ist; und gegebenenfalls umfaßt

(d) eine folgende Hydrolyse der aus der Stufe (c) resultierenden Verbindung, worin $R^3$ Cyano ist, unter Bildung der Verbindung, worin $R^3$ Carboxy ist.

9. Verfahren nach Anspruch 8, bei dem das N des Pyridinrings in der $\beta$-Stellung vorliegt.

10. Pharmazeutische Zusammensetzung, enthaltend einen pharmazeutischen Träger und eine wirksame Menge einer Verbindung nach Anspruch 1 oder eines pharmazeutisch brauchbaren Salzes davon.

**Revendications**

1. Composé de formule:

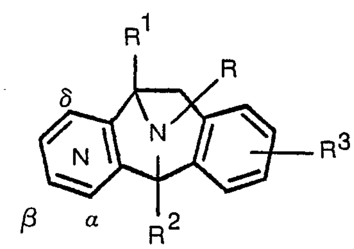

ou

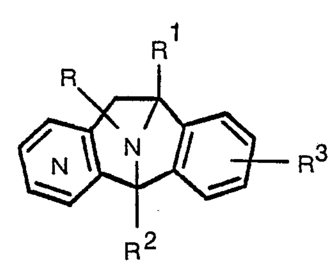

ou un sel d'un tel composé acceptable pour l'usage pharmaceutique, dans lesquels:
$R^1$ et $R^2$ représentent indépendamment
(1) l'hydrogène,
(2) un groupe alkyle en $C_1$—$C_5$,
(3) un groupe alcényle en $C_2$—$C_5$,
(4) un groupe phénylakyle contenant 1 à 3 atomes de carbone dans la partie alkyle,
(5) un groupe cycloalkyle en $C_3$—$C_6$,
(6) un groupe cycloalkylalkyle contenant 3 à 6 atomes de carbone dans la partie cycloalkyle, et 1 à 3 atomes de carbone dans la partie alkyle,
R représente
(1) $R^1$,
(2) un groupe phényle ou bien
(3) un groupe di-(alkyle en $C_1$—$C_3$)-amino-alkyle en $C_1$—$C_5$ et
$R^3$ représente
(1) l'hydrogène,
(2) un halogène,
(3) un groupe alcoxy en $C_1$—$C_5$,
(4) un groupe trifluorométhylthio,
(5) un groupe cyano ou bien
(6) un groupe carboxy.

2. Composé selon revendication 1, ou un sel d'un tel composé acceptable pour l'usage pharmaceutique,
dans lesquels:
$R^1$ et $R^2$ représentent indépendamment
(1) l'hydrogène,
(2) un groupe méthyle ou éthyle,
(3) un groupe vinyle ou allyle,
(4) un groupe benzyle,
(5) un groupe cyclopropyle ou cyclohexyle,
(6) un groupe cycloalkylalkyle contenant 3 à 6 atomes de carbone dans la partie cycloalkyle et 1 à 3 atomes de carbone dans la partie alkyle,
R représente
(1) $R^1$,
(2) un groupe phényle ou bien
(3) un groupe di-(alkyle en $C_1$—$C_3$)-amino-alkyle en $C_1$—$C_5$ et
$R^3$ représente
(1) l'hydrogène,
(2) le chlore, le brome, le fluor ou l'iode;
(3) un groupe méthoxy,
(4) un groupe trifluorométhylthio,
(5) un groupe cyano ou
(6) un groupe carboxy.

3. Composé selon la revendication 1 ou 2 dans lequel l'azote du noyau de pyridine est en position alpha, bêta ou delta.

4. Composé selon la revendication 3, dans lequel
$R^3$ représente l'hydrogène,
$R^1$ représente l'hydrogène, et
R et $R^2$ représentent indépendamment l'hydrogène, des groupes alkyles en $C_1$—$C_5$ ou benzyles.

5. Composé selon la revendication 4, de formule:

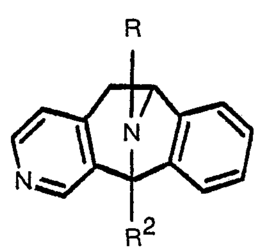

dans laquelle:
R représente l'hydrogène, ou groupe alkyle en $C_1$—$C_5$ ou benzyle, et
$R^2$ représente l'hydrogène ou un groupe alkyle en $C_1$—$C_5$.

6. Procédé pour la préparation d'un composé selon la revendication 1, qui comprend: le traitement d'un composé de formule:

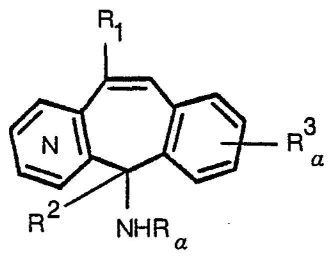

ou

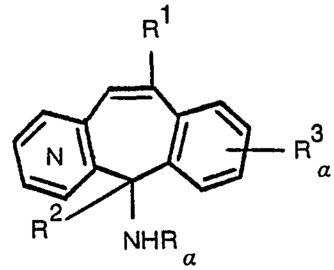

dans laquelle:

$R_\alpha$ représente R, un groupe alcanoyle inférieur, phénylsulfonyle, toluène-sulfonyle, alcoxycarbonyle, diphénylphosphinyle ou arylsulfonyle, et

$R^3_\alpha$ représente l'hydrogène ou un halogène,

par un acide, ce qui donne le composé de formule:

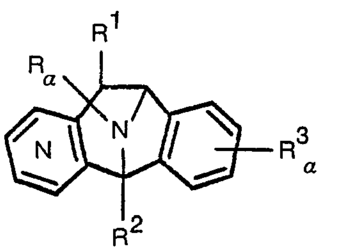

ou

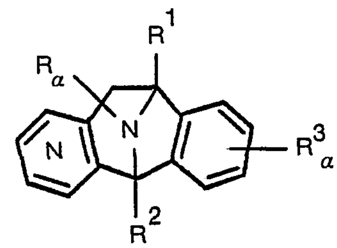

suivi de l'hydrolyse ou de l'hydrogénolyse de $R_\alpha$ lorsque $R_\alpha$ a une signification autre que R, et facultativement d'un traitement ultérieur des composés dans lesquels $R^3$ représente le brome,

(a) soit par un alcoolate de métal alcalin correspondant, ce qui donne le composé dans lequel $R^3$ représente un groupe alcoxy en $C_1$—$C_5$,

(b) soit par le trifluorométhylthio-cuivre, ce qui donne le composé dans lequel $R^3$ représente le groupe trifluorométhyle,

(c) soit par le cyanure cuivreux, ce qui donne le composé dans lequel $R^3$ représente un groupe cyano, et facultativement,

(d) d'une hydrolyse subséquente du composé obtenu au stade c), dans lequel $R^3$ représente le groupe cyano, ce qui donne le composé dans lequel $R^3$ représente le groupe carboxy.

7. Procédé pour la préparation d'un composé de formule:

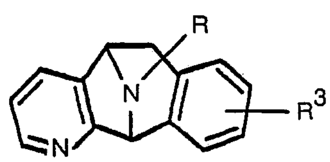

dans laquelle:

(1) l'hydrogène,

(2) un groupe alkyle en $C_1$—$C_5$,

(3) un groupe alcényle en $C_2$—$C_5$,

(4) un groupe phénylalkyle contenant 1 à 3 atomes de carbone dans la partie alkyle,

(5) un groupe cycloalkyle en $C_3$—$C_6$,

(6) un groupe cycloalkylalkyle contenant 3 à 6 atomes de carbone dans la partie cycloalkyle et 1 à 3 atomes de carbone dans la partie alkyle, ou bien

(7) un groupe di-(alkyle en $C_1$—$C_3$)-amino-alkyle en $C_1$—$C_5$ et

$R^3$ a les significations indequées dans la revendication 1, qui comprend l'hydrogénolyse des groupes X d'un composé de formule:

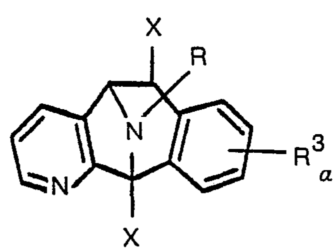

dans laquelle:

X représente Br ou Cl, et
$R^3_\alpha$ représente l'hydrogène ou un halogène,
ce qui donne un composé de formule:

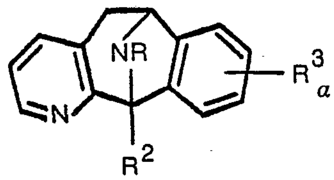

et facultativement un traitement ultérieur du composé dans lequel $R^3_\alpha$ représente le brome,

(a) soit par un alcoolate de métal alcalin correspondant, ce qui donne le composé dans lequel $R^3$ représente un groupe alcoxy en $C_1$—$C_5$,

(b) soit par le trifluorométhylthio-cuivre, ce qui donne le composé dans lequel $R^3$ représente le groupe trifluorométhyle,

(c) soit par le cyanure cuivreux, ce qui donne le composé dans lequel $R^3$ représente un groupe cyano, et facultativement

(d) une hydrolyse subséquente des composés du stade c) dans lesquels $R^3$ représente un groupe cyano, ce qui donne le composé dans lequel $R^3$ représente le groupe carboxy.

8. Procédé de préparation d'un composé de formule:

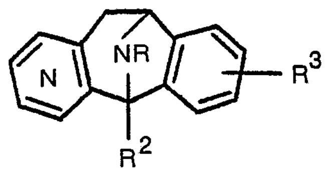

dans laquelle R, $R^2$ et $R^3$ ont les significations indiquées dans la revendication 1, qui comrend

(a) une réduction d'un composé de formule:

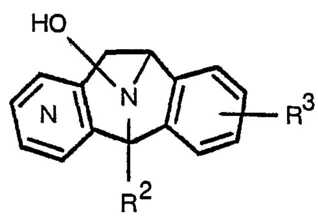

ce qui donne le composé dans lequel R représente l'hydrogène, suivie facultativement de

(b) une introduction du radical R représentant un groupe alkyle en $C_1$—$C_5$, un groupe alcényle en $C_2$—$C_5$, un groupe phénylalkyle contenant 1 à 3 atomes de carbone dans la partie alkyle, un groupe cycloalkyle en $C_3$—$C_6$, un groupe cycloalkylalkyle contenant 3 à 6 atomes de carbone dans la partie cycloalkyle et 1 à 3 atomes de carbone dans la partie alkyle, un groupe phényle ou un groupe di-(alkyle en $C_1$—$C_3$)-amino-alkyle en $C_1$—$C_5$ dans les composés de formule:

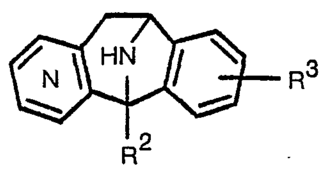

par un traitement de N-alkylation, ce qui donne le composé dans lequel R a la signification indiquée ci-dessus, et/ou facultativement,

(c) un traitement du composé dans lequel $R^3$ représente le brome par un alcoolate de sodium ou de potassium correspondant, par la cyanure cuivreux ou par le bis-(trifluorométhylthio)-mercure et la poudre de cuivre, ce qui donne les composés dans lesquels $R^3$ représente un groupe alcoxy en $C_1$—$C_5$, cyano ou trifluorométhylthio respectivement; et facultativement

(d) une hydrolyse subséquente du composé obtenu au stade c) et dans lequel $R^3$ représente un groupe cyano, ce qui donne le composé dans lequel $R^3$ représente un groupe carboxy.

9. Procédé selon la revendication 8, dans lequel l'azote du noyau de pyridine est en position bêta.

10. Composition pharmaceutique comprenant un véhicule pharmaceutique et une quantité efficace d'un composé selon la revendication 1 ou d'un sel d'un tel composé acceptable pour l'usage pharmaceutique.